# EUROPEAN PATENT APPLICATION

(11) **EP 3 878 515 A1**
(43) Date of publication of application: **15.09.2021**
(21) Application number: 20161922.8
(22) Date of filing: 09.03.2020
(51) Int. Cl.: A61P 35/00, C07K 14/31, C07K 14/315

(54) **THERAPEUTIC AGENT TARGETING HER2**

(71) Applicant: Hober Biotech AB, 115 46 Stockholm (SE)
(72) Inventor: HOBER, Sofia, 115 46 Stockholm (SE); WITTING, Emma von, 12 149 Johanneshov (SE); GAROUSI, Javad, 75 421 Uppsala (SE); TOLMACHEV, Vladimir, 75 266 Uppsala (SE)
(74) Representative: Kransell & Wennborg KB

(57) **Abstract**

There is provided a therapeutic conjugate comprising a fusion protein and a cytotoxic radionuclide, which cytotoxic radionuclide is bound to the fusion protein. The fusion protein comprises a certain HER2-binding region (HBR), a certain albumin-binding region (ABR) and a spacer region.

SEQUENCE LISTING

<110>  Hober Biotech AB

<120>  THERAPEUTIC AGENT

<130>  200064EP

<160>  35

<170>  PatentIn version 3.5

<210>  1
<211>  46
<212>  PRT
<213>  Homo sapiens

<400>  1

Leu Ala Glu Ala Lys Val Leu Ala Asn Arg Glu Leu Asp Lys Tyr Gly
1               5                   10                  15

Val Ser Asp Phe Tyr Lys Arg Leu Ile Asn Lys Ala Lys Thr Val Glu
            20                  25                  30

Gly Val Glu Ala Leu Lys Leu His Ile Leu Ala Ala Leu Pro
        35                  40                  45

<210>  2
<211>  46
<212>  PRT
<213>  Homo sapiens

<400>  2

Leu Ala Glu Ala Lys Glu Ala Ala Asn Ala Glu Leu Asp Ser Tyr Gly
1               5                   10                  15

Val Ser Asp Phe Tyr Lys Arg Leu Ile Asp Lys Ala Lys Thr Val Glu
            20                  25                  30

Gly Val Glu Ala Leu Lys Asp Ala Ile Leu Ala Ala Leu Pro
        35                  40                  45

<210>  3
<211>  47
<212>  PRT
<213>  Homo sapiens

<400>  3

Gly Leu Ala Glu Ala Lys Glu Ala Ala Asn Ala Glu Leu Asp Ser Tyr
1               5                   10                  15

Gly Val Ser Asp Phe Tyr Lys Arg Leu Ile Asp Lys Ala Lys Thr Val
            20                  25                  30

Glu Gly Val Glu Ala Leu Lys Asp Ala Ile Leu Ala Ala Leu Pro
        35                  40                  45

<210>  4
<211>  46
<212>  PRT
<213>  Homo sapiens

<400>  4

Leu Ala Glu Ala Lys Val Leu Ala Asn Arg Glu Leu Asp Lys Tyr Gly
1               5                   10                  15

Val Ser Asp Tyr Tyr Lys Asn Leu Ile Asn Asn Ala Lys Thr Val Glu
            20                  25                  30

Gly Val Lys Ala Leu Ile Asp Glu Ile Leu Ala Ala Leu Pro
        35                  40                  45

<210>  5
<211>  46
<212>  PRT
<213>  Homo sapiens

<400>  5

Leu Ala Glu Ala Lys Val Leu Ala Leu Arg Glu Leu Asp Lys Tyr Gly
1               5                   10                  15

Val Ser Asp Tyr Tyr Lys Asp Leu Ile Asp Lys Ala Lys Thr Val Glu
            20                  25                  30

Gly Val Lys Ala Leu Ile Asp Glu Ile Leu Ala Ala Leu Pro
        35                  40                  45

<210>  6
<211>  46
<212>  PRT
<213>  Homo sapiens

<400>  6

Leu Ala Ala Ala Lys Glu Thr Ala Leu Tyr His Leu Asp Arg Leu Gly
1               5                   10                  15

Val Ala Asp Ala Tyr Lys Asp Leu Ile Asp Lys Ala Lys Thr Val Glu
            20                  25                  30

Gly Val Lys Ala Arg Tyr Phe Glu Ile Leu His Ala Leu Pro
        35                  40                  45

<210>  7
<211>  46
<212>  PRT
<213>  Homo sapiens

<400>  7

Leu Ala Ala Ala Lys Glu Thr Ala Leu Tyr His Leu Asp Arg Val Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Leu Glu Ile Leu Pro Ala Leu Pro
        35                  40                  45

<210>  8
<211>  46
<212>  PRT
<213>  Homo sapiens

<400>  8

Leu Ala Pro Ala Lys Glu Thr Ala Leu Tyr His Leu Asp Arg Val Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Phe Glu Ile Leu His Ala Leu Pro
        35                  40                  45

<210>  9
<211>  46
<212>  PRT
<213>  Homo sapiens

<400>  9

Leu Ala Ala Ala Lys Glu Thr Ala Leu Tyr His Leu Asp Arg Leu Gly
1               5                   10                  15

Val Ser Asp Tyr Tyr Lys Asp Leu Ile Asp Lys Ala Lys Thr Val Glu
            20                  25                  30

Gly Val Lys Ala Leu Tyr Phe Glu Ile Leu His Ala Leu Pro
        35                  40                  45

<210>  10
<211>  46
<212>  PRT
<213>  Homo sapiens

<400>  10

Leu Ala Pro Ala Lys Glu Thr Ala Leu Tyr His Leu Asp Arg Leu Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Leu Glu Ile Leu Lys Ala Leu Pro
        35                  40                  45

<210>  11
<211>  46
<212>  PRT
<213>  Homo sapiens

<400>  11

Leu Ala Gly Ala Lys Glu Thr Ala Leu Tyr His Leu Asp Arg Leu Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Leu Glu Ile Leu Thr Ala Leu Pro
        35                  40                  45

<210>  12
<211>  46
<212>  PRT
<213>  Homo sapiens

<400>  12

Leu Ala Pro Ala Lys Glu Thr Ala Leu Tyr His Leu Asp Arg Leu Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Phe Glu Ile Leu Arg Ala Leu Pro
        35                  40                  45

<210>  13
<211>  46
<212>  PRT
<213>  Homo sapiens

<400>  13

Leu Ala Gly Ala Lys Glu Thr Ala Leu Tyr His Leu Asp Arg Val Gly
1               5                   10                  15

Val Ser Asp Tyr Tyr Lys Asp Leu Ile Asp Lys Ala Lys Thr Val Glu

20                  25                  30

Gly Val Arg Ala Leu Tyr Leu Glu Ile Leu Arg Ala Leu Pro
        35                  40                  45

<210>  14
<211>  46
<212>  PRT
<213>  Homo sapiens

<400>  14

Leu Ala Ala Ala Lys Glu Thr Ala Leu Tyr His Leu Asp Arg Val Gly
1               5                   10                  15

Val Ser Asp Tyr Tyr Lys Asp Leu Ile Asp Lys Ala Lys Thr Val Glu
            20                  25                  30

Gly Val Met Ala Leu Tyr Ala Glu Ile Leu Pro Ala Leu Pro
        35                  40                  45

<210>  15
<211>  46
<212>  PRT
<213>  Homo sapiens

<400>  15

Leu Ala Gly Ala Lys Glu Thr Ala Leu Tyr His Leu Asp Lys Thr Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Leu Glu Ile Leu Gln Ala Leu Pro
        35                  40                  45

<210>  16
<211>  46
<212>  PRT
<213>  Homo sapiens

<400>  16

Leu Ala Ala Ala Lys Glu Thr Ala Leu Tyr His Leu Thr Arg Val Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Phe Glu Ile Leu Tyr Ala Leu Pro
        35                  40                  45

<210>  17
<211>  46
<212>  PRT
<213>  Homo sapiens

<400>  17

Leu Ala Ser Ala Lys Asp Thr Ala Leu Tyr His Leu Asp Arg Val Gly
1               5                   10                  15

Val Ser Asp Tyr Tyr Lys Asp Leu Ile Asp Lys Ala Lys Thr Val Glu
            20                  25                  30

Gly Val Arg Ala Leu Tyr Ala Glu Ile Leu Ala Ala Leu Pro
        35                  40                  45

<210>  18
<211>  10
<212>  PRT
<213>  Homo sapiens

<400>  18

Met Gly Asp Glu Ala Val Asp Ala Asn Ser
1               5                   10

<210>  19
<211>  46
<212>  PRT
<213>  Homo sapiens

<400>  19

Leu Ala Glu Ala Lys Val Leu Ala Asn Arg Glu Leu Asp Lys Tyr Gly
1               5                   10                  15

Val Ala Asp Ala Tyr Lys Asn Leu Ile Asn Asn Ala Lys Thr Val Glu
            20                  25                  30

Gly Val Lys Ala Arg Ile Asp Glu Ile Leu Ala Ala Leu Pro
        35                  40                  45

<210>  20
<211>  118
<212>  PRT
<213>  Homo sapiens

<400>  20

Met Gly Asp Glu Ala Val Asp Ala Asn Ser Leu Ala Glu Ala Lys Val
1               5                   10                  15

Leu Ala Asn Arg Glu Leu Asp Lys Tyr Gly Val Ala Asp Ala Tyr Lys
            20                  25                  30

Asn Leu Ile Asn Asn Ala Lys Thr Val Glu Gly Val Lys Ala Arg Ile
        35                  40                  45

Asp Glu Ile Leu Ala Ala Leu Pro Ser Ser Ser Gly Ser Ser Ser Gly
    50                  55                  60

Ser Ser Ser Gly Leu Ala Glu Ala Lys Val Leu Ala Asn Arg Glu Leu
65                  70                  75                  80

Asp Lys Tyr Gly Val Ser Asp Phe Tyr Lys Arg Leu Ile Asn Lys Ala
                85                  90                  95

Lys Thr Val Glu Gly Val Glu Ala Leu Lys Leu His Ile Leu Ala Ala
            100                 105                 110

Leu Pro Gly Ser Ser Cys
        115

<210>  21
<211>  104
<212>  PRT
<213>  Homo sapiens

<400>  21

Leu Ala Ala Ala Lys Glu Thr Ala Leu Tyr His Leu Asp Arg Leu Gly
1               5                   10                  15

Val Ala Asp Ala Tyr Lys Asp Leu Ile Asp Lys Ala Lys Thr Val Glu
            20                  25                  30

Gly Val Lys Ala Arg Tyr Phe Glu Ile Leu His Ala Leu Pro Ser Ser
        35                  40                  45

Ser Gly Ser Ser Ser Gly Ser Ser Ser Gly Leu Ala Glu Ala Lys Val
    50                  55                  60

Leu Ala Asn Arg Glu Leu Asp Lys Tyr Gly Val Ser Asp Phe Tyr Lys
65                  70                  75                  80

Arg Leu Ile Asn Lys Ala Lys Thr Val Glu Gly Val Glu Ala Leu Lys
                85                  90                  95

Leu His Ile Leu Ala Ala Leu Pro
            100

<210>  22
<211>  104

<212>  PRT
<213>  Homo sapiens

<400>  22

Leu Ala Glu Ala Lys Val Leu Ala Asn Arg Glu Leu Asp Lys Tyr Gly
1               5                   10                  15

Val Ser Asp Phe Tyr Lys Arg Leu Ile Asn Lys Ala Lys Thr Val Glu
            20                  25                  30

Gly Val Glu Ala Leu Lys Leu His Ile Leu Ala Ala Leu Pro Ser Ser
        35                  40                  45

Ser Gly Ser Ser Ser Gly Ser Ser Ser Gly Leu Ala Ala Ala Lys Glu
    50                  55                  60

Thr Ala Leu Tyr His Leu Asp Arg Leu Gly Val Ala Asp Ala Tyr Lys
65                  70                  75                  80

Asp Leu Ile Asp Lys Ala Lys Thr Val Glu Gly Val Lys Ala Arg Tyr
                85                  90                  95

Phe Glu Ile Leu His Ala Leu Pro
            100


<210>  23
<211>  112
<212>  PRT
<213>  Homo sapiens

<400>  23

Leu Ala Ala Ala Lys Glu Thr Ala Leu Tyr His Leu Asp Arg Leu Gly
1               5                   10                  15

Val Ala Asp Ala Tyr Lys Asp Leu Ile Asp Lys Ala Lys Thr Val Glu
            20                  25                  30

Gly Val Lys Ala Arg Tyr Phe Glu Ile Leu His Ala Leu Pro Ser Ser
        35                  40                  45

Ser Gly Ser Ser Ser Gly Ser Ser Ser Gly Ser Ser Ser Gly Ser Ser
    50                  55                  60

Ser Gly Leu Ala Glu Ala Lys Val Leu Ala Asn Arg Glu Leu Asp Lys
65                  70                  75                  80

Tyr Gly Val Ser Asp Phe Tyr Lys Arg Leu Ile Asn Lys Ala Lys Thr
                85                  90                  95

Val Glu Gly Val Glu Ala Leu Lys Leu His Ile Leu Ala Ala Leu Pro
            100                 105                 110

<210>  24
<211>  112
<212>  PRT
<213>  Homo sapiens

<400>  24

Leu Ala Glu Ala Lys Val Leu Ala Asn Arg Glu Leu Asp Lys Tyr Gly
1               5                   10                  15

Val Ser Asp Phe Tyr Lys Arg Leu Ile Asn Lys Ala Lys Thr Val Glu
            20                  25                  30

Gly Val Glu Ala Leu Lys Leu His Ile Leu Ala Ala Leu Pro Ser Ser
        35                  40                  45

Ser Gly Ser Ser Ser Gly Ser Ser Ser Gly Ser Ser Ser Gly Ser Ser
    50                  55                  60

Ser Gly Leu Ala Ala Ala Lys Glu Thr Ala Leu Tyr His Leu Asp Arg
65                  70                  75                  80

Leu Gly Val Ala Asp Ala Tyr Lys Asp Leu Ile Asp Lys Ala Lys Thr
                85                  90                  95

Val Glu Gly Val Lys Ala Arg Tyr Phe Glu Ile Leu His Ala Leu Pro
            100                 105                 110

<210>  25
<211>  8
<212>  PRT
<213>  Homo sapiens

<400>  25

Asp Glu Ala Val Asp Ala Asn Ser
1               5

<210>  26
<211>  60
<212>  PRT
<213>  Homo sapiens

<400>  26

Met Gly Asp Glu Ala Val Asp Ala Asn Ser Leu Ala Ala Ala Lys Glu
1               5                   10                  15

Thr Ala Leu Tyr His Leu Asp Arg Leu Gly Val Ala Asp Ala Tyr Lys

20                  25                  30

Asp Leu Ile Asp Lys Ala Lys Thr Val Glu Gly Val Lys Ala Arg Tyr
        35                  40                  45

Phe Glu Ile Leu His Ala Leu Pro Gly Ser Ser Cys
    50                  55                  60

<210>  27
<211>  118
<212>  PRT
<213>  Homo sapiens

<400>  27

Met Gly Asp Glu Ala Val Asp Ala Asn Ser Leu Ala Ala Ala Lys Glu
1               5                   10                  15

Thr Ala Leu Tyr His Leu Asp Arg Leu Gly Val Ala Asp Ala Tyr Lys
            20                  25                  30

Asp Leu Ile Asp Lys Ala Lys Thr Val Glu Gly Val Lys Ala Arg Tyr
        35                  40                  45

Phe Glu Ile Leu His Ala Leu Pro Ser Ser Ser Gly Ser Ser Ser Gly
    50                  55                  60

Ser Ser Ser Gly Leu Ala Glu Ala Lys Val Leu Ala Asn Arg Glu Leu
65                  70                  75                  80

Asp Lys Tyr Gly Val Ser Asp Phe Tyr Lys Arg Leu Ile Asn Lys Ala
                85                  90                  95

Lys Thr Val Glu Gly Val Glu Ala Leu Lys Leu His Ile Leu Ala Ala
            100                 105                 110

Leu Pro Gly Ser Ser Cys
        115

<210>  28
<211>  118
<212>  PRT
<213>  Homo sapiens

<400>  28

Met Gly Asp Glu Ala Val Asp Ala Asn Ser Leu Ala Glu Ala Lys Val
1               5                   10                  15

Leu Ala Asn Arg Glu Leu Asp Lys Tyr Gly Val Ser Asp Phe Tyr Lys
            20                  25                  30

Arg Leu Ile Asn Lys Ala Lys Thr Val Glu Gly Val Glu Ala Leu Lys
        35                  40                  45

Leu His Ile Leu Ala Ala Leu Pro Ser Ser Ser Gly Ser Ser Ser Gly
    50                  55                  60

Ser Ser Ser Gly Leu Ala Ala Ala Lys Glu Thr Ala Leu Tyr His Leu
65                  70                  75                  80

Asp Arg Leu Gly Val Ala Asp Ala Tyr Lys Asp Leu Ile Asp Lys Ala
                85                  90                  95

Lys Thr Val Glu Gly Val Lys Ala Arg Tyr Phe Glu Ile Leu His Ala
            100                 105                 110

Leu Pro Gly Ser Ser Cys
        115

<210>  29
<211>  126
<212>  PRT
<213>  Homo sapiens

<400>  29

Met Gly Asp Glu Ala Val Asp Ala Asn Ser Leu Ala Ala Ala Lys Glu
1               5                   10                  15

Thr Ala Leu Tyr His Leu Asp Arg Leu Gly Val Ala Asp Ala Tyr Lys
            20                  25                  30

Asp Leu Ile Asp Lys Ala Lys Thr Val Glu Gly Val Lys Ala Arg Tyr
        35                  40                  45

Phe Glu Ile Leu His Ala Leu Pro Ser Ser Ser Gly Ser Ser Ser Gly
    50                  55                  60

Ser Ser Ser Gly Ser Ser Ser Gly Ser Ser Ser Gly Leu Ala Glu Ala
65                  70                  75                  80

Lys Val Leu Ala Asn Arg Glu Leu Asp Lys Tyr Gly Val Ser Asp Phe
                85                  90                  95

Tyr Lys Arg Leu Ile Asn Lys Ala Lys Thr Val Glu Gly Val Glu Ala
            100                 105                 110

Leu Lys Leu His Ile Leu Ala Ala Leu Pro Gly Ser Ser Cys
        115                 120                 125

<210>  30
<211>  126
<212>  PRT
<213>  Homo sapiens

<400>  30

Met Gly Asp Glu Ala Val Asp Ala Asn Ser Leu Ala Glu Ala Lys Val
1               5                   10                  15

Leu Ala Asn Arg Glu Leu Asp Lys Tyr Gly Val Ser Asp Phe Tyr Lys
            20                  25                  30

Arg Leu Ile Asn Lys Ala Lys Thr Val Glu Gly Val Glu Ala Leu Lys
        35                  40                  45

Leu His Ile Leu Ala Ala Leu Pro Ser Ser Ser Gly Ser Ser Ser Gly
    50                  55                  60

Ser Ser Ser Gly Ser Ser Ser Gly Ser Ser Ser Gly Leu Ala Ala Ala
65                  70                  75                  80

Lys Glu Thr Ala Leu Tyr His Leu Asp Arg Leu Gly Val Ala Asp Ala
                85                  90                  95

Tyr Lys Asp Leu Ile Asp Lys Ala Lys Thr Val Glu Gly Val Lys Ala
            100                 105                 110

Arg Tyr Phe Glu Ile Leu His Ala Leu Pro Gly Ser Ser Cys
        115                 120                 125


<210>  31
<211>  8
<212>  PRT
<213>  Homo sapiens

<400>  31

Ser Ser Ser Gly Ser Ser Ser Gly
1               5


<210>  32
<211>  12
<212>  PRT
<213>  Homo sapiens

<400>  32

Ser Ser Ser Gly Ser Ser Ser Gly Ser Ser Ser Gly
1               5                   10

<210>  33
<211>  16
<212>  PRT
<213>  Homo sapiens

<400>  33

Ser Ser Ser Gly Ser Ser Ser Gly Ser Ser Ser Gly Ser Ser Ser Gly
1               5                   10                  15


<210>  34
<211>  20
<212>  PRT
<213>  Homo sapiens

<400>  34

Ser Ser Ser Gly Ser Ser Ser Gly Ser Ser Ser Gly Ser Ser Ser Gly
1               5                   10                  15


Ser Ser Ser Gly
            20


<210>  35
<211>  24
<212>  PRT
<213>  Homo sapiens

<400>  35

Ser Ser Ser Gly Ser Ser Ser Gly Ser Ser Ser Gly Ser Ser Ser Gly
1               5                   10                  15


Ser Ser Ser Gly Ser Ser Ser Gly
            20

## Description

### TECHNICAL FIELD

The present disclosure relates to the field of HER2-targeted therapy.

### BACKGROUND

Engineered scaffold proteins (ESPs) attract an increasing interest as specific targeting vectors in radionuclide molecular imaging (Krasniqi 2018). ESPs have a number of attractive features such as efficient and inexpensive production in prokaryotic hosts or even by peptide synthesis. Moreover, they show high stability in a broad range of temperature and pH, facilitating the use of a large variety of labeling methods. In addition, their high stability and mutational tolerance promotes the ease of engineering multimeric, multispecific and multifunctional constructs. "ADAPT" is an ESP derived from the small (46 amino acids) albumin-binding domain (ABD) of protein G (Nilvebrant Hober S 2013). Feasibility of using ADAPTs as imaging probes has been demonstrated using ADAPT₆, which shows high affinity to human epidermal growth factor receptor type 2 (HER2) (Garousi 2015). However, the use of ADAPT₆-based radionuclide therapy has been precluded by high renal reabsorption after clearance via glomerular filtration. The re-absorption cannot be reduced by co-injection of L-lysine or Gelofusine (Lindbo 2016). Therefore, other strategies are needed to allow for therapeutic use.

### SUMMARY

The present inventors have realized that a non-covalent binding to serum albumin is a possible strategy to extend circulatory half-life, increase bioavailability and reduce renal uptake of small therapeutics undergoing renal clearance.

An objective of the present disclosure is to provide a HER2-targeting therapeutic agent that overcomes at least some of the shortcomings of the prior art.

To meet this objective, there is provided a therapeutic conjugate comprising a fusion protein and a cytotoxic radionuclide, which cytotoxic radionuclide is bound to the fusion protein, wherein:
- the fusion protein comprises a HER2-binding region (HBR), an albumin-binding region (ABR) and a spacer region;
- the number of amino acids of the spacer region is at least 7, such as 7-30;
- the HBR comprises an amino acid sequence selected from
   i) LAX₃AKX₆TX₈X₉YHLX₁₃X₁₄X₁₅GVX₁₈DX₂₀YKX₂₃LIDKX₂₈KT VEX₃₃VX₃₅AX₃₇YX₃₉X₄₀ILX₄₃ALP (SEQ ID NO:36), wherein, independently of each other,
      X₃ is selected from A, G, P, S and V;
      X₆ is selected from D and E;
      X₈ is selected from A and V;
      X₉ is selected from L and N;
      X₁₃ is selected from D and T;
      X₁₄ is selected from K and R;
      X₁₅ is selected from I, L, M, T and V;
      X₁₈ is selected from S and A;
      X₂₀ is selected from F, Y and A;
      X₂₃ is selected from D and R;
      X₂₈ is selected from A and V;
      X₃₃ is selected from G, S and D;
      X₃₅ is selected from K, M and R;
      X₃₇ is selected from L and R;
      X₃₉ is selected from A, F and L;
      X₄₀ is selected from A and E; and
      X₄₃ is selected from A, H, K, P, R, T, Q and Y;
   and ii) an amino acid sequence which has at least 95% identity to the sequence defined in i); and
- the ABR comprises an amino acid sequence selected from
   a) LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILX₄₃X₄₄LP (SEQ ID NO:37), wherein, independently of each other,
      X₃ is selected from E, S, Q and C;
      X₆ is selected from E, S, V and C;
      X₇ is selected from A, L and S;
      X₉ is selected from L and N;
      X₁₀ is selected from A, S and R;
      X₁₄ is selected from A, S, C and K;
      X₂₀ is selected from Y and F;
      X₂₃ is selected from N, D and R;
      X₂₆ is selected from N, D and E;
      X₂₇ is selected from N and K;
      X₃₅ is selected from K and E;
      X₃₈ is selected from I and K;
      X₃₉ is selected from D, E and L;
      X₄₀ is selected from A, E and H;
      X₄₃ is selected from A and K;
      X₄₄ is selected from A, S and E;
      L in position 45 is present or absent; and
      P in position 46 is present or absent;
   and b) an amino acid sequence which has at least 95% identity to the sequence defined in a).

In an embodiment, the cytotoxic radionuclide is coupled to a terminal end (preferably the C-terminal end) of the fusion protein, typically by chelator-based conjugation or covalent conjugation.

In an embodiment, the spacer region connects the C-terminal end of the HBR to the N-terminal end of the ABR.

In an embodiment, the ABR comprises the amino acid sequence LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILAALP (SEQ ID NO:38), wherein, independently of each other,
X₃ is selected from E and S;
X₆ is selected from E and V;
X₇ is selected from A and L;
X₉ is selected from L and N;
X₁₀ is selected from A and R;
X₁₄ is selected from A, S, C and K, preferably from A, S and K;
X₂₀ is selected from Y and F;
X₂₃ is selected from N, D and R;
X₂₆ is selected from N and D;
X₂₇ is selected from N and K;
X₃₅ is selected from K and E;
X₃₈ is selected from I and K;
X₃₉ is selected from D and L;
X₄₀ is selected from A, E and H;
L in position 45 is present or absent; and
P in position 46 is present or absent.

In an embodiment, the ABR comprises the amino acid sequence LAEAKX₆X₇ANX₁₀ ELDX₁₄YGVSDF YKRLIX₂₆KAKT VEGVEALKX₃₉X₄₀ ILAALP (SEQ ID NO:39), wherein, independently of each other,
X₆ is selected from E and V;
X₇ is selected from A and L;
X₁₀ is selected from A and R;
X₁₄ is selected from S and K;
X₂₆ is selected from N and D;
X₃₉ is selected from D and L; and
X₄₀ is selected from A and H.

In an embodiment, the ABR comprises or consists of an amino acid sequence selected from the group consisting of: 
LAEAKVLANR ELDKYGVSDF YKRLINKAKT VEGVEALKLH ILAALP (SEQ ID NO:1);
LAEAKEAANA ELDSYGVSDF YKRLIDKAKT VEGVEALKDA ILAALP (SEQ ID NO:2);
GLAEAKEAAN AELDSYGVSD FYKRLIDKAK TVEGVEALKD AILAALP (SEQ ID NO:3);
LAEAKVLANR ELDKYGVSDY YKNLINNAKT VEGVKALIDE ILAALP (SEQ ID NO:4);
 and
LAEAKVLALR ELDKYGVSDY YKDLIDKAKT VEGVKALIDE ILAALP (SEQ ID NO:5).

In an embodiment of amino acid sequence i):
X₃ is selected from A, G, P;
X₆ is E;
X₉ is L;
X₁₃ is D;
X₁₄ is R;
X₁₅ is selected from L and V;
X₁₈ is selected from S and A;
X₂₀ is selected from F, Y and A;
X₂₈ is A;
X₃₃ is G;
X₃₅ is selected from K and R;
X₃₇ is L;
X₃₉ is selected from F and L;
X₄₀ is E; and
X₄₃ is selected from H, P and R.

In an embodiment, the HBR comprises or consists of an amino acid sequence selected from the group consisting of: 
LAAAKETALY HLDRLGVADA YKDLIDKAKT VEGVKARYFE ILHALP (SEQ ID NO:6);
LAAAKETALY HLDRVGVSDY YKDLIDKAKT VEGVRALYLE ILPALP (SEQ ID NO:7);
LAPAKETALY HLDRVGVSDY YKDLIDKAK TVEGVRALYFE ILHALP (SEQ ID NO:8);
LAAAKETALY HLDRLGVSDY YKDLIDKAK TVEGVKALYFE ILHALP (SEQ ID NO:9);
LAPAKETALY HLDRLGVSDY YKDLIDKAK TVEGVRALYLE ILKALP (SEQ ID NO:10);
LAGAKETALY HLDRLGVSDY YKDLIDKAK TVEGVRALYLE ILTALP (SEQ ID NO:11);
LAPAKETALY HLDRLGVSDY YKDLIDKAK TVEGVRALYFE ILRALP (SEQ ID NO:12);
LAGAKETALY HLDRVGVSDY YKDLIDKAK TVEGVRALYLE ILRALP (SEQ ID NO:13);
LAAAKETALY HLDRVGVSDY YKDLIDKAK TVEGVMALYAE ILPALP (SEQ ID NO:14);
LAGAKETALY HLDKTGVSDY YKDLIDKAK TVEGVRALYLE ILQALP (SEQ ID NO:15);
LAAAKETALY HLTRVGVSDY YKDLIDKAK TVEGVRALYFE ILYALP (SEQ ID NO:16);
 and
LASAKDTALY HLDRVGVSDYYKDLIDKAK TVEGVRALYAE ILAALP (SEQ ID NO:17).

In an embodiment, the cytotoxic radionuclide is selected from the group consisting of ¹⁷⁷Lu, ⁹⁰Y, ¹⁸⁸Re; ¹⁸⁶Re; ¹⁶⁶Ho, ¹⁵³Sm, ⁶⁷Cu, ⁶⁴Cu, ¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.

In an embodiment, the group of cytotoxic radionuclides is limited to ¹⁷⁷Lu, ⁹⁰Y, ¹³¹I, ¹⁵³Sm, ²²⁵Ac; ¹⁸⁸Re and ¹⁸⁶Re.

In an embodiment, the radionuclide is a radiometal and the fusion protein is conjugated to the radiometal by a chelator that is covalently bound to the fusion protein, preferably to a cysteine (C) residue of the fusion protein. Accordingly, an embodiment of the fusion protein further comprises a terminal cysteine (C) residue, preferably a C-terminal cysteine (C) residue. The terminal cysteine (C) residue is preferably the only cysteine (C) residue of the fusion protein.

In an embodiment, the number of amino acids of the spacer region is at least 8, such as 8-30, such as 8-20, preferably 8-14, more preferably 10-14.

In an embodiment, the spacer region comprises a repeat of the amino acid sequence SSSG.

In an embodiment, the therapeutic conjugate comprises no more than 150 amino acid residues, such as no more than 130 amino acid residues.

In an embodiment, the fusion protein further comprises an additional region comprising the amino acid sequence DEAVDANS (SEQ ID NO:25). In such an embodiment, the additional region may extend from the N-terminal end of the HBR.

The present disclosure further provides a pharmaceutical composition comprising the above-mentioned therapeutic conjugate and a pharmaceutically acceptable carrier.

The present disclosure also provides a therapeutic conjugate according to the above for use in a method of treating a cancer, typically a cancer overexpressing HER2. The cancer type may be a breast cancer or a gastric/gastroesophageal cancer.

The therapeutic conjugate of the present disclosure exhibits excellent biodistribution, which means that side effects are limited during treatment of patients having HER2-expressing tumors.

As explained above, the therapeutic conjugate of the present disclosure comprises a fusion protein having an albumin-binding region (ABR) and a HER2-binding region (HBR), which are linked to each other by a spacer region. The function of the spacer region is to enable free refolding and prevent steric hindrance of binding to albumin and HER2.

### BRIEF DESCRIPTION OF THE DRAWINGS

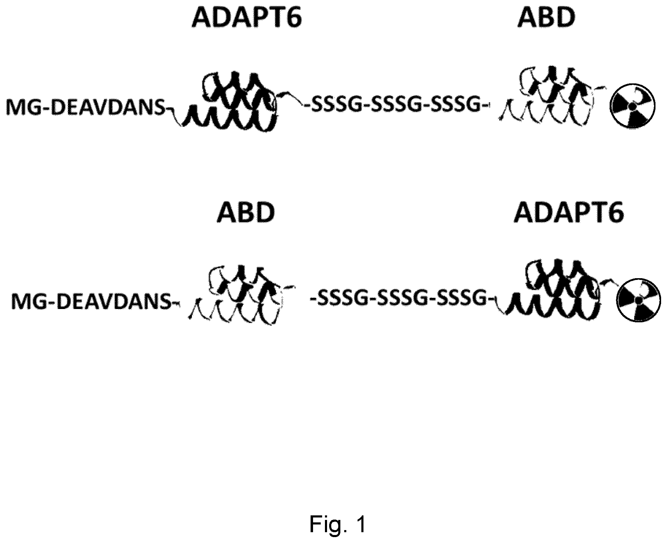

Figure 1 illustrates two examples of therapeutic conjugates according to the present disclosure.

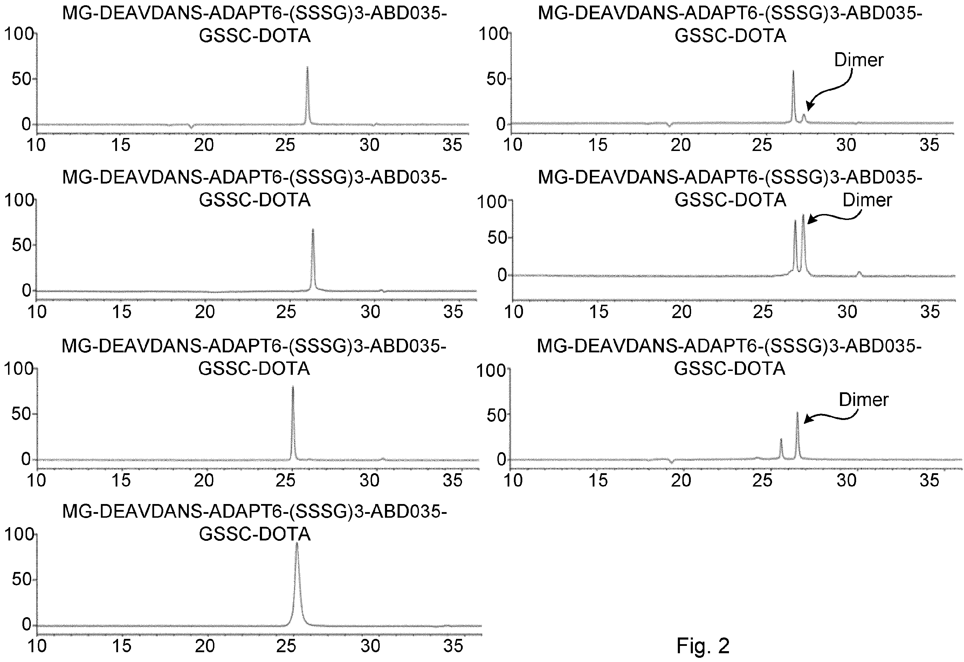

Figure 2 shows analytical HPLC chromatograms showing the purity of the different protein variants. The right column contains unconjugated protein intended for ¹²⁵I labelling and hence shows up as two separate peaks corresponding to the monomer and the dimer formed by the free cysteine in non-reducing conditions. In the chromatograms of Figure 2, the X axis represents time [minutes] and the Y axis represents mAU.

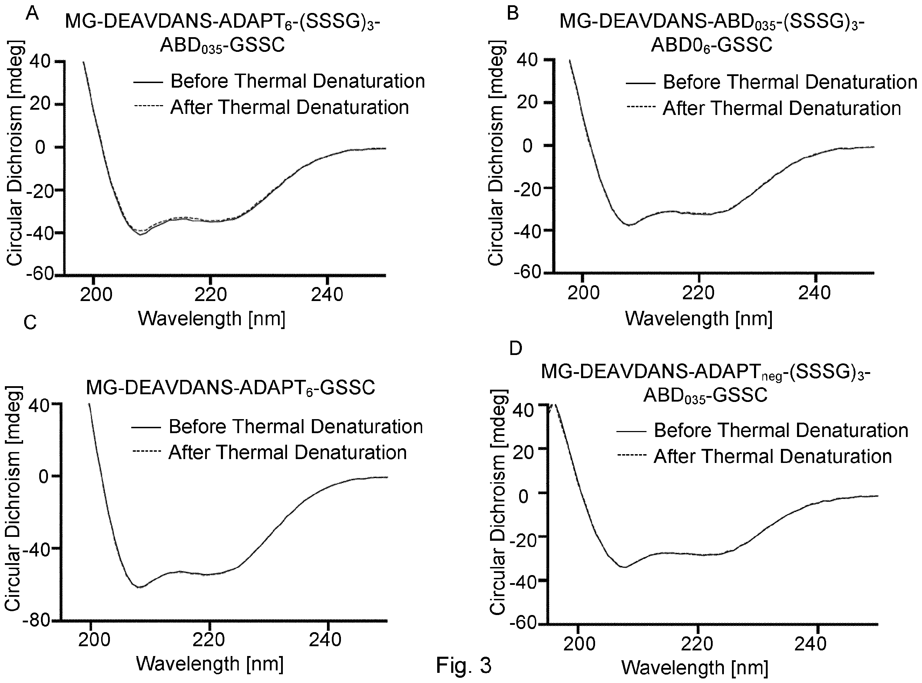

Figure 3 shows CD spectra of the proteins used in this study, demonstrating high alpha helical content as well as ability to refold after thermal denaturation. In the spectra of Figure 3, the X axis represents wavelength [nm] and the Y axis circular dichroism [mdeg].

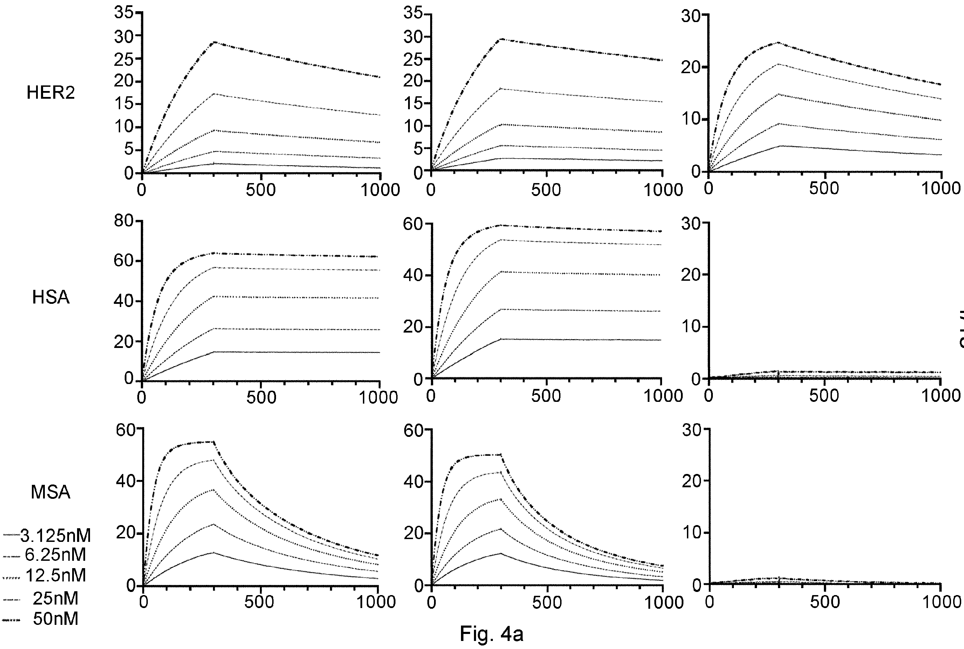

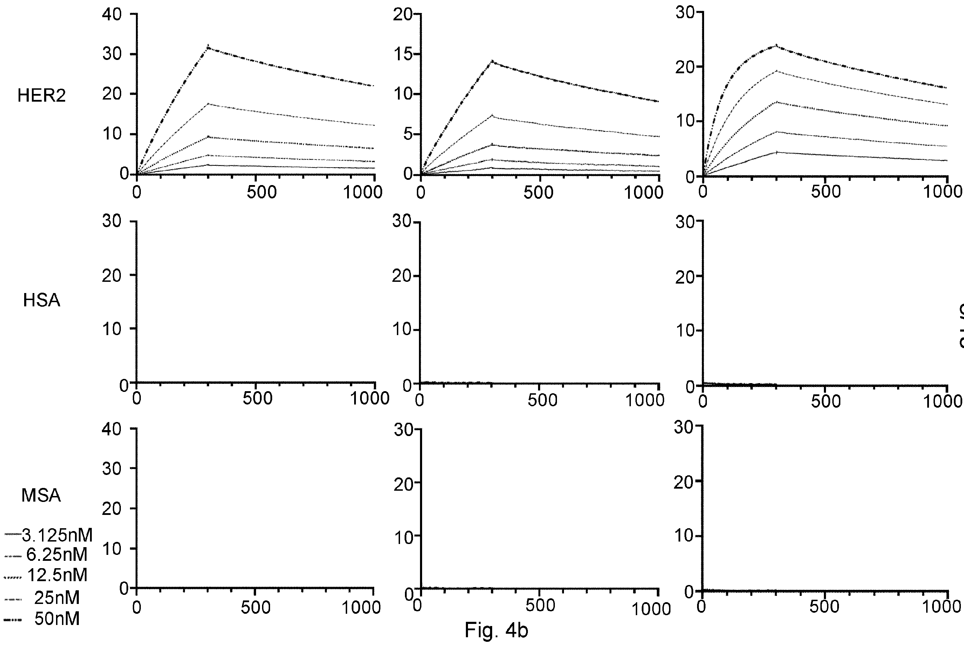

Figure 4 shows SPR sensorgrams of the targeting proteins binding to HER2, Human Serum Albumin (HSA) and Murine Serum Albumin (MSA) in a concentration dependent manner. Figure 4b rows show binding in a surrounding of saturating concentrations of HSA (analyte saturated with 10x excess HSA). Hence, simultaneous binding of the fusion proteins to HER2 and HSA is demonstrated by exhibiting binding to HER2 even after saturation with HSA. However, the binding to HSA and MSA, respectively, while having saturating concentrations of HSA in the buffer is not possible due to blocking of the binding site. The left-hand column represents MG-DEAVDANS-ADAPT₆-(SSSG)₃-ABD₀₃₅-GSSC. The middle column represents MG-DEAVDANS- ABD₀₃₅-(SSSG)₃-ADAPT₆-GSSC. The right-hand column represents MG-DEAVDANS-ADAPT₆-GSSC. In the graphs of Figure 4, the X axis represents time [s] and the Y axis represents Response Unit [RU].

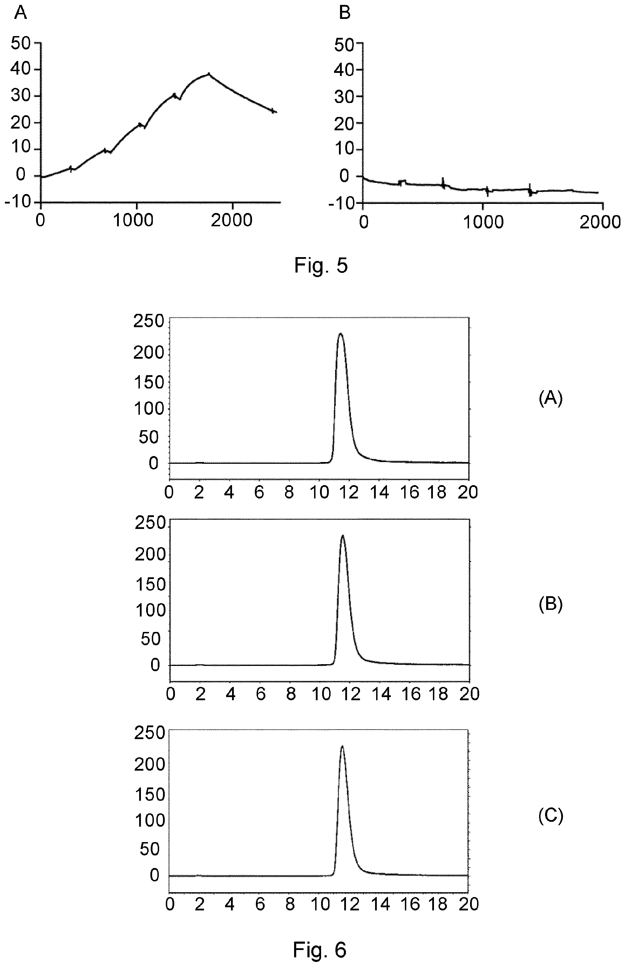

Figure 5A shows SPR sensorgram of the targeting fusion protein (MG-DEAVDANS-ADAPT₆-(SSSG)₃-ABD₀₃₅-GSSC) used for the therapy study. Figure 5B shows SPR sensorgram of the non-targeting control fusion protein (MG-DEAVDANS-ADAPT_{neg}-(SSSG)₃-ABD₀₃₅-GSSC). The absence of binding curvature in Figure 5B confirms that ADAPT_{neg} does not bind HER2. In the graphs of Figure 5, the X axis represents time [s] and the Y axis represents Response Unit [RU].
Figure 6 shows RadioHPLC chromatograms of MG-DEAVDANS-ADAPT₆-GSSC-HPEM-¹²⁵I (A), MG-DEAVDANS-ABD₀₃₅-(SSSG-ADAPT₆-GSSC-HPEM-¹²⁵I (B) and MG-DEAVDANS-ADAPT₆-(SSSG)₃-ABD₀₃₅-GSSC-HPEM-¹²⁵I (C). In the chromatograms of Figure 6, the X axis represents time [Minutes] and the Y axis represents mV.

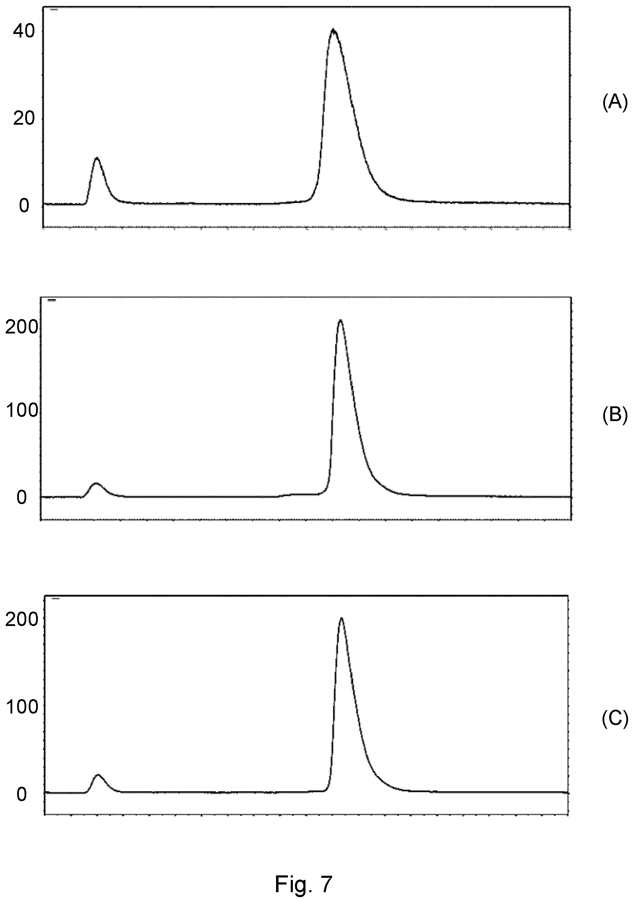

Figure 7 shows RadioHPLC chromatograms of MG-DEAVDANS-ADAPT₆-GSSC-DOTA-¹⁷⁷Lu (A), MG-DEAVDANS-ABD₀₃₅-(SSSG)₃-ADAPT₆-GSSC-DOTA-¹⁷⁷Lu (B) and MG-DEAVDANS-ADAPT₆-(SSSG)₃-ABD₀₃₅-GSSC-DOTA-¹⁷⁷Lu (C). In the chromatograms of Figure 6, the X axis represents time [Minutes] and the Y axis represents mV.

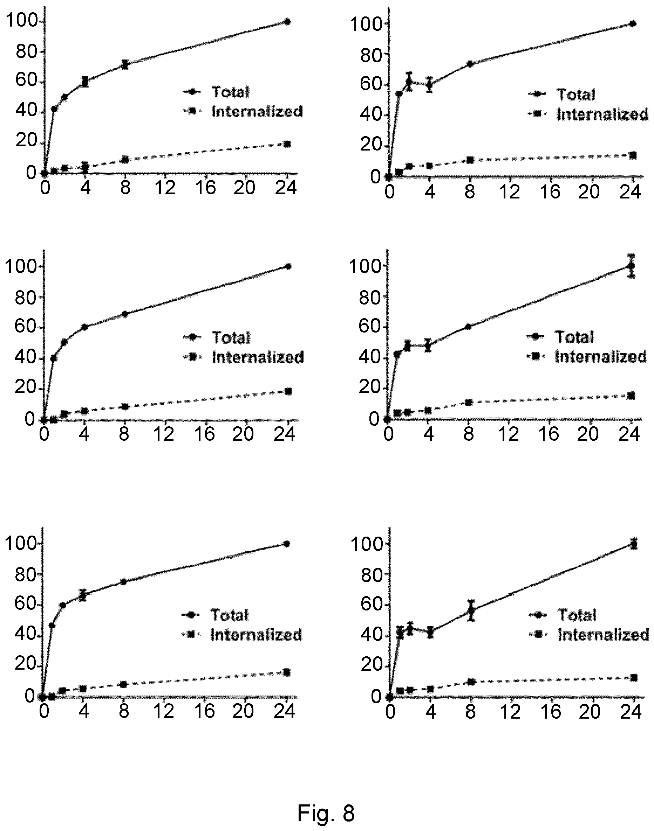

Figure 8 shows cellular processing of MG-DEAVDANS-ADAPT₆-GSSC-DOTA-¹⁷⁷Lu (first row), MG-DEAVDANS-ABD₀₃₅-(SSSG)₃-ADAPT₆-GSSC-DOTA-¹⁷⁷Lu (second row) and MG-DEAVDANS-ADAPT₆-(SSSG)₃-ABD₀₃₅-GSSC-DOTA-¹⁷⁷Lu (third row) by HER2-expressing SKOV-3 (left column) and BT-474 (right column) cell lines. Data are normalized to a maximum average cell-associated activity for each conjugate. Data are presented as mean values with standard deviations from three cell dishes. In the graphs of Figure 8, the X axis represents time [h] and the Y axis represents normalized cell-associated radioactivity (%).

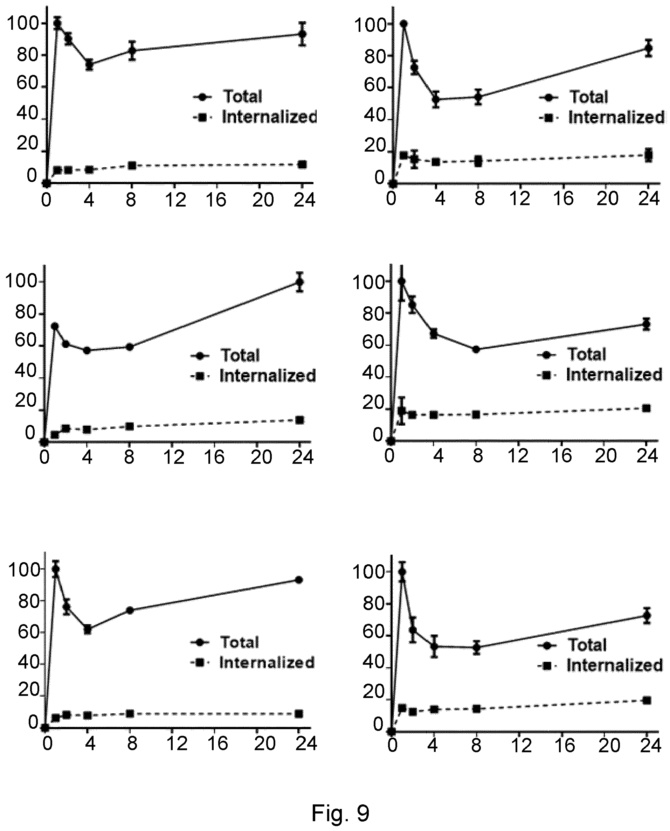

Figure 9 shows cellular processing of MG-DEAVDANS-ADAPT₆-GSSC-HPEM-¹²⁵I (first row), MG-DEAVDANS-ABD₀₃₅-(SSSG)₃-ADAPT₆-GSSC-HPEM-¹²⁵I (second row) and MG-DEAVDANS-ADAPT₆-(SSSG)₃-ABD₀₃₅-GSSC-HPEM-¹²⁵I (third row) by HER2-expressing SKOV-3 (left column) and BT-474 (right column) cell lines. Data are normalized to a maximum average cell-associated activity for each conjugate. Data are presented as mean values with standard deviations from three cell dishes. In the graphs of Figure 9, the X axis represents time [h] and the Y axis represents normalized cell-associated radioactivity (%).

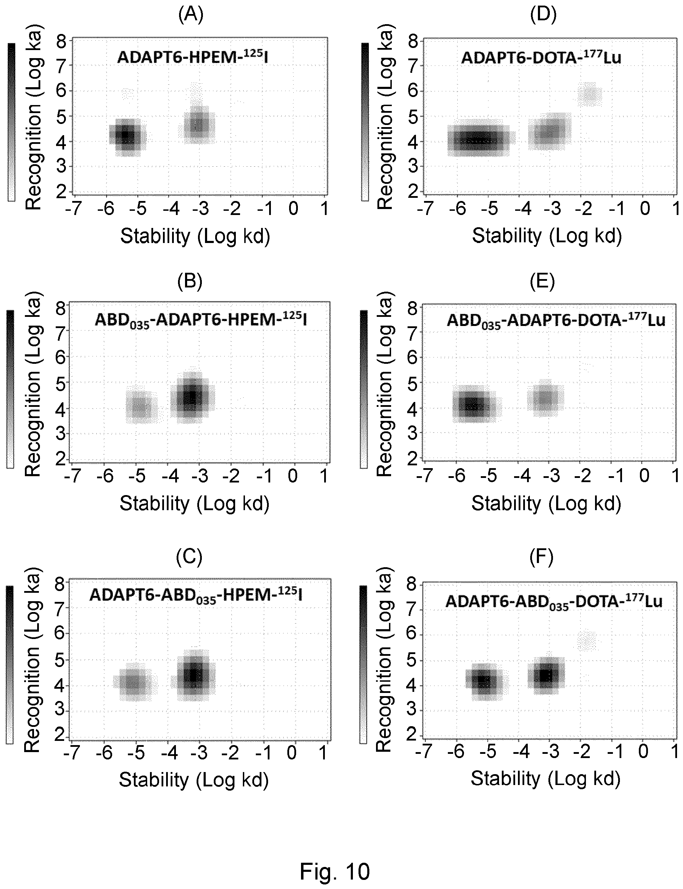

Figure 10 shows an InteractionMap of binding of radiolabeled conjugates to SKOV-3 cells. The radiolabeled conjugates are MG-DEAVDANS-ADAPT₆-GSSC-HPEM-¹²⁵I (A), MG-DEAVDANS-ABD₀₃₅-(SSSG)₃-ADAPT₆-GSSC-HPEM-¹²⁵I (B), MG-DEAVDANS-ADAPT₆-(SSSG)₃-ABD₀₃₅-GSSC-HPEM-¹²⁵I (C), MG-DEAVDANS-ADAPT₆-GSSC-DOTA-¹⁷⁷Lu (D), MG-DEAVDANS-ABD₀₃₅-(SSSG)₃-ADAPT₆-GSSC-DOTA-¹⁷⁷Lu (E) and MG-DEAVDANS-ADAPT₆-(SSSG)₃-ABD₀₃₅-GSSC-DOTA-¹⁷⁷Lu (F).

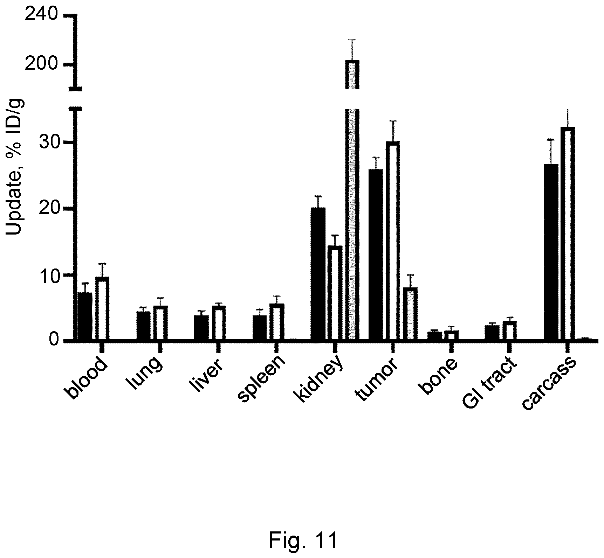

Figure 11 shows the effect of ABD fusion on biodistribution of ¹⁷⁷Lu-labelled ADAPT variants. The biodistribution in BALB/C nu/nu mice bearing SKOV-3 xenografts was measured at 48 h after injection. Data are expressed as %ID/g and are averages from 5 animals ± SD. The filled/black bars represent MG-DEAVDANS-ABD₀₃₅-(SSSG)₃-ADAPT₆-GSSC-DOTA-¹⁷⁷Lu, the empty bars represent MG-DEAVDANS-ADAPT₆-(SSSG)₃-ABD₀₃₅-GSSC-DOTA-¹⁷⁷Lu and the grey bars represent MG-DEAVDANS-ADAPT₆-GSSC-DOTA-¹⁷⁷Lu (reference). Notably, the grey bar reaches 200%ID/g in kidney.

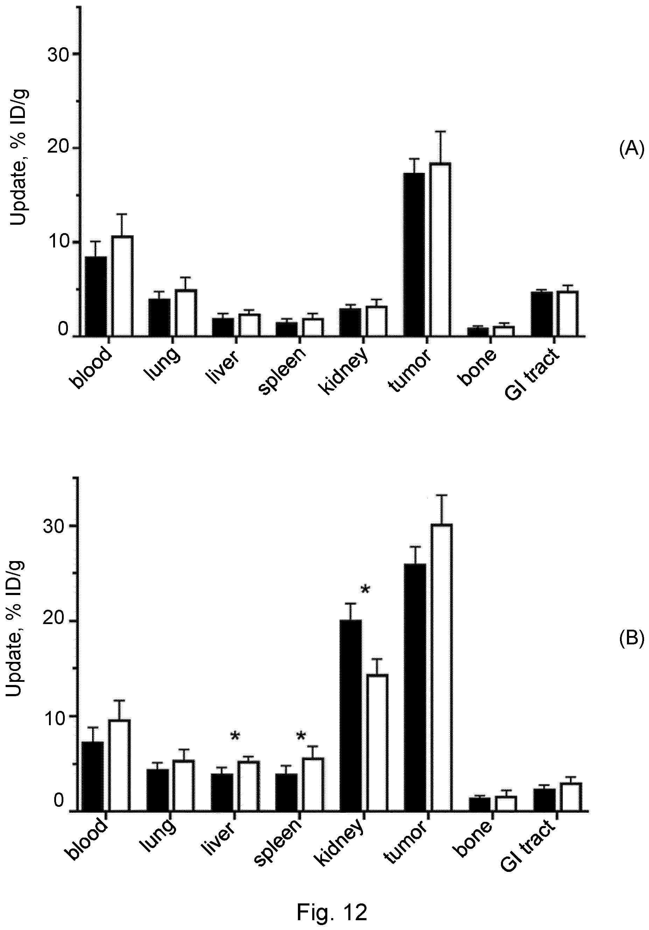

Figure 12 shows a comparison between ¹²⁵I-labelled (A) and ¹⁷⁷Lu-labelled (B) ABD-fused ADAPT variants. The biodistribution in BALB/C nu/nu mice bearing SKOV-3 xenografts was measured at 48 h after injection. Data are expressed as %ID/g and are averages from 5 animals ± SD. Asterisk marks significant difference in uptake. In Figure 12A, empty bars represent MG-DEAVDANS-ADAPT₆-(SSSG)₃₋ABD₀₃₅-GSSC-HPEM-¹²⁵I and filled bars represent MG-DEAVDANS-ABD₀₃₅-(SSSG)₃-ADAPT₆-GSSC-HPEM-¹²⁵I. In Figure 12B, empty bars represent MG-DEAVDANS-ADAPT₆-(SSSG)₃-ABD₀₃₅-GSSC-DOTA-¹⁷⁷Lu and filled bars represent MG-DEAVDANS-ABD₀₃₅-(SSSG)₃-ADAPT₆-GSSC-DOTA-¹⁷⁷Lu.

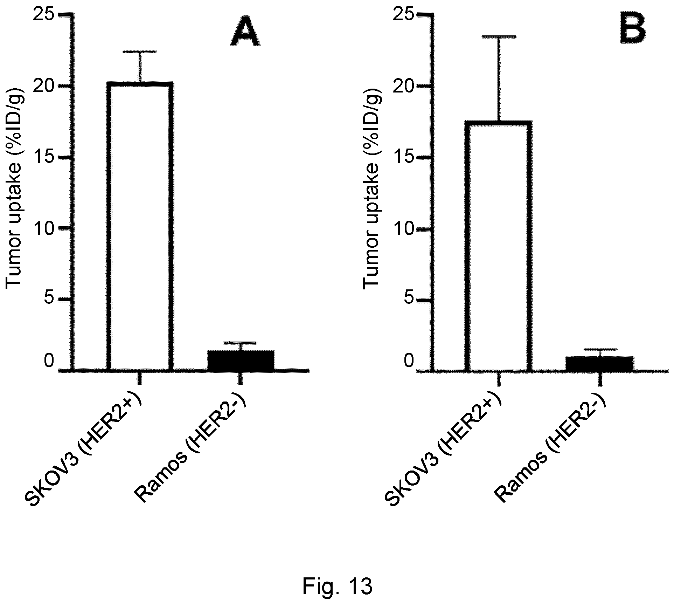

Figure 13 shows in vivo tumor uptake specificity of MG-DEAVDANS-ADAPT6-(SSSG)₃-ABD₀₃₅-GSSC-HPEM-¹²⁵I (A) and MG-DEAVDANS-ADAPT6-(SSSG)₃-ABD₀₃₅-GSSC-DOTA-¹⁷⁷Lu (B) 48 h after injection. Uptake in HER2-positive SKOV-3 and HER2-negative Ramos xenograft is compared. Data are expressed as %ID/g and are averages from 4 animals ± SD.

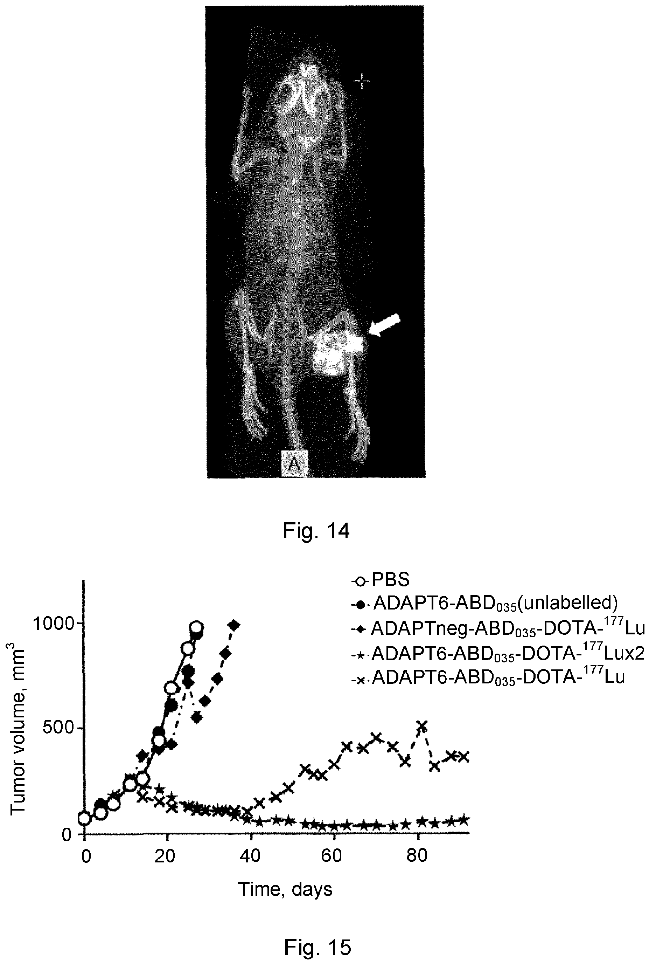

Figure 14 shows a SPECT/CT image of MG-DEAVDANS-ADAPT6-(SSSG)₃-ABD₀₃₅-GSSC-DOTA-¹⁷⁷Lu distribution in a mouse bearing HER2-positive SKOV-3 tumor xenograft 72 h after injection. The arrow in the figure points at the highest uptake, which is in the tumor.
Figure 15 shows average tumor volumes of mice treated with: PBS; non-labelled MG-DEAVDANS-ADAPT₆-(SSSG)₃-ABD₀₃₅-GSSC-DOTA; single injection of 18 MBqMG-DEAVDANS-ADAPT₆-(SSSG)₃-ABD₀₃₅-GSSC-DOTA-¹⁷⁷Lu; double injection of 18 MBqMG-DEAVDANS-ADAPT₆-(SSSG)₃-ABD₀₃₅-GSSC-DOTA-¹⁷⁷Lu; and single injection of 18 MBq MG-DEAVDANS-ADAPT_{Neg}-(SSSG)₃-ABD₀₃₅-GSSC-DOTA-¹⁷⁷Lu.

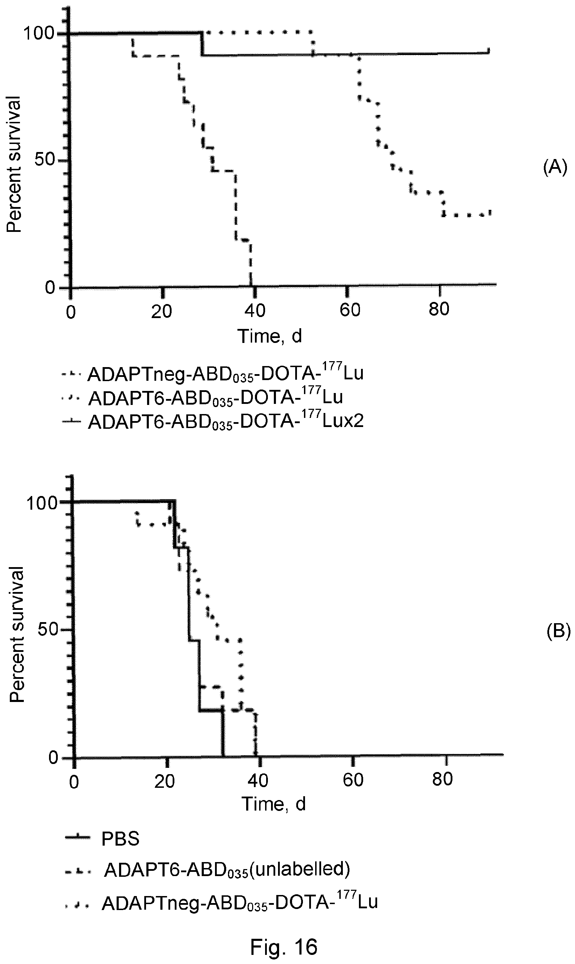

Figure 16 shows results from in vivo therapy. Survival of BALB/c nude mice (n=9-10) bearing HER2-expressing SKOV-3 xenografts after injection(s) of: PBS; non-labeled MG-DEAVDANS-ADAPT₆-(SSSG)₃-ABD₀₃₅-GSSC-DOTA; 18 MBq MG-DEAVDANS-ADAPT₆-(SSSG)₃-ABD₀₃₅-GSSC-DOTA-¹⁷⁷Lu (single injection); 18 MBq MG-DEAVDANS-ADAPT₆-(SSSG)₃-ABD₀₃₅-GSSC-DOTA-¹⁷⁷Lu (double injection); and 18 MBq MG-DEAVDANS-ADAPT_{Neg}-(SSSG)₃-ABD₀₃₅-GSSC-DOTA-¹⁷⁷Lu (single injection).
In Figure 16A:
   MG-DEAVDANS-ADAPT_{Neg}-(SSSG)₃-ABD₀₃₅-GSSC-DOTA-¹⁷⁷Lu (single injection) is represented by a dashed line;
   MG-DEAVDANS-ADAPT₆-(SSSG)₃-ABD₀₃₅-GSSC-DOTA-¹⁷⁷Lu (single injection) is represented by a dotted line; and
   MG-DEAVDANS-ADAPT₆-(SSSG)₃-ABD₀₃₅-GSSC-DOTA-¹⁷⁷Lu (double injection) is represented by a solid line.
In Figure 16B:
   MG-DEAVDANS-ADAPT_{Neg}-(SSSG)₃-ABD₀₃₅-GSSC-DOTA-¹⁷⁷Lu (single injection) is represented by a dotted line;
   MG-DEAVDANS-ADAPT₆-(SSSG)₃-ABD₀₃₅-GSSC-DOTA (unlabelled) is represented by a dotted line; and
   PBS is represented by a solid line.

### DETAILED DESCRIPTION

ADAPT6 has shown great promise in both pre-clinical and clinical imaging applications mainly because of its small size and fast blood clearance. However, the small size and hence the substantial kidney uptake also prevents further therapeutic use. In the present disclosure, half-life extension strategy where the protein of interest is fused to a sequence based on the Albumin Binding Domain (ABD) of Protein G has been utilized, to take advantage of the long half-life of the patient's own serum albumin.

Our molecular design of HBR-ABR-based fusion proteins permits production of probes capable of correct folding and high-affinity binding to both albumin and HER2 (Table 1). The ability of the fusion proteins to refold after thermal denaturation has allowed the use of high temperatures for labeling, e.g. with ¹⁷⁷Lu, which should ensure high coupling stability (Price 2014). Indeed, the label was stable under challenge with a large molar excess of EDTA, and binding specificity and affinity of ¹⁷⁷Lu-labeled fusion proteins was not compromised (Table 4). Cellular processing study with the use of residualizing ¹⁷⁷Lu label suggested that both MG-DEAVDANS-ABD₀₃₅-(SSSG)₃-ADAPT₆-GSSC-DOTA-¹⁷⁷Lu and MG-DEAVDANS-ADAPT₆-(SSSG)₃-ABD₀₃₅-GSSC-DOTA-¹⁷⁷Lu internalized slowly, to the same extent as a non-fused ADAPT6 (Figure 8). This might indicate that residualizing properties of a label are less critical for retention of a label by cancer cells. Therefore, variants with non-residualizing ¹²⁵I-HPEM label, MG-DEAVDANS-ABD₀₃₅-(SSSG)₃-ADAPT6-GSSC-HPEM-¹²⁵I and MG-DEAVDANS-ADAPT6-(SSSG)₃-ABD₀₃₅-GSSC-HPEM-¹²⁵I, were also included in the evaluation. As is customary in the field, ¹²⁵I was used as a surrogate for ¹³¹I, which is used in the clinic.

An *in vivo* targeting experiment (Figure 11) showed an appreciably enhanced retention of activity of ABD-fused ADAPTs in blood and reduction of their uptake in kidneys in comparison with non-fused ADAPT. It has to be noted that although fusion of targeting proteins with ABD has been applied earlier, the extent of such effect is unpredictable due to complexity of interactions of all constituents of a construct *in vivo.* For example, fusing of ABD with ADAPT6-targeted polypeptide toxins had much more modest effect on retention in blood and uptake in kidneys (Liu 2019).

Unexpectedly, the present disclosure shows that the relative positioning of ABD₀₃₅ (the ABR) and ADAPT6 (the HBR) had an effect, in particular in case of using ¹⁷⁷Lu as the cytotoxic radionuclide. Connecting the ABR to the C-terminal end of the HBR resulted not only in significantly higher tumor uptake, but also significantly lower kidney uptake. The fact that absorbed dose to kidneys might be dose limiting underlines the importance of these results.

The data presented herein demonstrates that both the ¹⁷⁷Lu-label and the ¹²⁵I-label were delivered to the tumor and that the delivery was more efficient in case of the ¹⁷⁷Lu-labelled fusion protein. Accumulation of both ¹⁷⁷Lu- and ¹²⁵I-labeled ADAPT6-ABD₀₃₅ in tumors was apparently dependent on HER2 expression (Figure 13). SPECT/CT imaging confirmed that uptake in tumor was much higher than in any other tissues at 72 h after injection (Figure 14). Dosimetry calculation suggested that the absorbed dose to tumor should be 67 Gy when keeping dose limits of 2 Gy for blood and 20 Gy to kidneys.

Results of an experimental therapy confirmed the dosimetry data. It is shown herein that control groups of mice treated with only PBS or unlabelled HBR-ABR (i.e. fusion protein lacking cytotoxic radionuclide) had a median survival of 25 days. Treatment with a radionuclide-labelled variant of the fusion protein that had been modified to remove the HER2-binding capacity caused slight but significant extension of median survival (31 d) (Figure 16B), most likely due to activity circulating in blood or taken up on tumors because of the EPR effect. When the intact version of the HBR was used, the effect on tumor growth (Figure 15 and 16A) was strikingly different. The tumor growth was suppressed in a dose-dependent manner and median survival was enhanced more than two-fold (70 days) even in the case of single injection. Importantly, the treatment was not associated with any observable toxicity. For example, the average animal weight did not differ significantly between treatment and control groups. Histopathology examinations of kidneys and livers excised at the end of treatment showed only subtle alterations in hepatocytes (rounded cytoplasmic vacuoles and increased variation in size and shape of the cell nucleus) and renal tubular cells (enlarged size of the cell nucleus and chromatin margination). These alterations were however reversible.

Currently, there are several treatment options for patients with disseminated HER2-expressing breast cancers, e.g. treatment with monoclonal antibodies trastuzumab and pertuzumab or antibody-drug conjugated trastuzumab-DMi. These therapeutics significantly improved survival of breast cancer patients. Unfortunately, tumors eventually develop resistance to such therapies. Remarkably, the resistant tumors preserve a high level of HER2 expression. Therapy using the therapeutic conjugate of the present disclosure is an additional option for such patients.

The present disclosure provides a therapeutic conjugate comprising a fusion protein and a cytotoxic radionuclide. The cytotoxic radionuclide is bound to the fusion protein.

In a preferred embodiment, the cytotoxic radionuclide is coupled to a terminal end of the fusion protein. Such coupling is typically achieved by chelator-based conjugation or covalent conjugation (further discussed below). In a particularly preferred embodiment, the cytotoxic radionuclide is coupled to the C-terminal end of the fusion protein.

The fusion protein comprises and an HER2-binding region (HBR), an albumin-binding region (ABR) and a spacer region. Preferably, the spacer region connects the C-terminal end of the HBR to the N-terminal end of the ABR. Advantages thereof is described in the Examples section below.

The number of amino acids of the spacer region is at least 7, such as 7-30. A spacer region that is relatively short, but still enables free refolding and prevents steric hindrance to binding to albumin and HER2 is preferred. Accordingly, the number of amino acids of the spacer region maybe 8-30, such as 8-20, preferably 8-14, more preferably 10-14.

As an example, the spacer region may comprise or consist of a repeat of the amino acid sequence SSSG. Consequently, the spacer region may comprise or consist of an amino acid sequence selected from the group consisting of SSSGSSSG ((SSSG)2, SEQ ID NO:31), SSSGSSSGSSSG ((SSSG)₃, SEQ ID NO:32), SSSGSSSGSSSGSSSG ((SSSG)₄, SEQ ID NO:33), SSSGSSSGSSSGSSSGSSSG ((SSSG)₅, SEQ ID NO:34) and SSSGSSSGSSSGSSSGSSSGSSSG ((SSSG)₆, SEQ ID NO:35). Preferably, the group consists of (SSSG)₂, (SSSG)₃ and (SSSG)₄.

The HBR comprises an amino acid sequence selected from
i) LAX₃AKX₆TX₈X₉YHLX₁₃X₁₄X₁₅GVX₁₈DX₂₀ YKX₂₃LIDKX₂₈KT VEX₃₃VX₃₅AX₃₇YX₃₉X₄₀ ILX₄₃ALP, wherein, independently of each other,
   X₃ is selected from A, G, P, S and V, preferably A, G and P;
   X₆ is selected from D and E, preferably E;
   X₈ is selected from A and V;
   X₉ is selected from L and N, preferably L;
   X₁₃ is selected from D and T, preferably D;
   X₁₄ is selected from K and R, preferably R;
   X₁₅ is selected from I, L, M, T and V, preferably L and V;
   X₁₈ is selected from S and A;
   X₂₀ is selected from F, Y and A;
   X₂₃ is selected from D and R;
   X₂₈ is selected from A and V, preferably A;
   X₃₃ is selected from G, S and D, preferably G;
   X₃₅ is selected from K, M and R, preferably K and R;
   X₃₇ is selected from L and R, preferably L;
   X₃₉ is selected from A, F and L, preferably F and L;
   X₄₀ is selected from A and E, preferably E; and
   X₄₃ is selected from A, H, K, P, R, T, Q and Y, preferably H, P and R;
and ii) an amino acid sequence which has at least 95% identity to the sequence defined in i).

Data showing binding activity of i) and ii) to HER2 is presented in WO2014076179, Nilvebrant 2014 and the Examples section below.

In preferred embodiment of the amino acid sequence i)
X₃ is selected from A, G, P, preferably A and G;
X₆ is E;
X₈ is A and V;
X₉ is L;
X₁₃ is D;
X₁₄ is R;
X₁₅ is selected from L and V;
X₁₈ is selected from S and A;
X₂₀ is selected from F, Y and A;
X₂₃ is selected from D and R;
X₂₈ is A;
X₃₃ is G;
X₃₅ is selected from K and R;
X₃₇ is L;
X₃₉ is selected from F and L;
X₄₀ is E; and
X₄₃ is selected from H, P and R.

In another preferred embodiment, the HBR comprises an amino acid sequence selected from the group consisting of: 
LAAAKETALY HLDRLGVADA YKDLIDKAKT VEGVKARYFE ILHALP (SEQ ID NO:6);
LAAAKETALY HLDRVGVSDY YKDLIDKAKT VEGVRALYLE ILPALP (SEQ ID NO:7);
LAPAKETALY HLDRVGVSDY YKDLIDKAK TVEGVRALYFE ILHALP (SEQ ID NO:8);
LAAAKETALY HLDRLGVSDY YKDLIDKAK TVEGVKALYFE ILHALP (SEQ ID NO:9);
LAPAKETALY HLDRLGVSDY YKDLIDKAK TVEGVRALYLE ILKALP (SEQ ID NO:10);
LAGAKETALY HLDRLGVSDY YKDLIDKAK TVEGVRALYLE ILTALP (SEQ ID NO:11);
LAPAKETALY HLDRLGVSDY YKDLIDKAK TVEGVRALYFE ILRALP (SEQ ID NO:12);
LAGAKETALY HLDRVGVSDY YKDLIDKAK TVEGVRALYLE ILRALP (SEQ ID NO:13);
LAAAKETALY HLDRVGVSDY YKDLIDKAK TVEGVMALYAE ILPALP (SEQ ID NO:14);
LAGAKETALY HLDKTGVSDY YKDLIDKAK TVEGVRALYLE ILQALP (SEQ ID NO:15);
LAAAKETALY HLTRVGVSDY YKDLIDKAK TVEGVRALYFE ILYALP (SEQ ID NO:16);
 and
LASAKDTALY HLDRVGVSDYYKDLIDKAK TVEGVRALYAE ILAALP (SEQ ID NO:17).

A particularly preferred group consists of SEQ ID NO:6, SEQ ID NO:9 and SEQ ID NO:13. SEQ ID NO:6 is used in the Examples section below. SEQ ID NO:9 and 13 were identified by both phage display and FACS in Nilvebrant 2014, which the inventors consider to be beneficial.

The ABR comprises an amino acid sequence selected from
a) LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILX₄₃X₄₄LP, wherein, independently of each other,
   X₃ is selected from E, S, Q and C, preferably E, S and Q;
   X₆ is selected from E, S, V and C, preferably E, S and V;
   X₇ is selected from A, L and S;
   X₉ is selected from L and N;
   X₁₀ is selected from A, S and R;
   X₁₄ is selected from A, S, C and K, preferably A, S and K;
   X₂₀ is selected from Y and F;
   X₂₃ is selected from N, D and R;
   X₂₆ is selected from N, D and E;
   X₂₇ is selected from N and K;
   X₃₅ is selected from K and E;
   X₃₈ is selected from I and K;
   X₃₉ is selected from D, E and L;
   X₄₀ is selected from A, E and H;
   X₄₃ is selected from A and K;
   X₄₄ is selected from A, S and E;
   L in position 45 is present or absent; and
   P in position 46 is present or absent;
and b) an amino acid sequence which has at least 95% identity to the sequence defined in a).

The scaffold of sequence a) is based on the wild type of the albumin binding domain (ABD_{wt}, SEQ ID NO:4) of protein G. Data showing binding activity of i) and ii) to serum albumin is presented in Jonsson 2008, WO2012004384 and the Examples section below.

In a preferred embodiment, the ABR comprises the amino acid sequence LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILAALP, wherein, independently of each other,
X₃ is selected from E and S;
X₆ is selected from E and V;
X₇ is selected from A and L;
X₉ is selected from L and N;
X₁₀ is selected from A and R;
X₁₄ is selected from A, S, C and K, preferably from A, S and K;
X₂₀ is selected from Y and F;
X₂₃ is selected from N, D and R;
X₂₆ is selected from N and D;
X₂₇ is selected from N and K;
X₃₅ is selected from K and E;
X₃₈ is selected from I and K;
X₃₉ is selected from D and L;
X₄₀ is selected from A, E and H;
L in position 45 is present or absent; and
P in position 46 is present or absent.

In a particularly preferred embodiment, the ABR comprises the amino acid sequence
LAEAKX₆X₇ANX₁₀ ELDX₁₄YGVSDF YKRLIX₂₆KAKT VEGVEALKX₃₉X₄₀ ILAALP, wherein, independently of each other,
X₆ is selected from E and V;
X₇ is selected from A and L;
X₁₀ is selected from A and R;
X₁₄ is selected from S and K;
X₂₆ is selected from N and D;
X₃₉ is selected from D and L; and
X₄₀ is selected from A and H.

This sequence covers ABD₃₅ and PEP07914 in WO2012004384. The reduced immune-stimulatory properties of the sequences disclosed in WO2012004384 are considered to be beneficial for the conjugate of the present disclosure. WO2012004384 has resulted in two granted European patents, EP2590993B1 and EP2933262B1. The ABR of the present disclosure may be defined as in these patents.

Consequently, the ABR may, as in EP2933262B1, comprise an amino acid sequence selected from
aa) LAX₃AKX₆X₇ANX₁₀ ELDX₁₄YGVSDF YKRLIX₂₆KAKT VEGVEALKX₃₉X₄₀ ILX₄₃X₄₄LP, wherein, independently of each other,
   X₃ is selected from E, S, Q and C, preferably E, S and Q;
   X₆ is selected from E, S and C, preferably E and S;
   X₇ is selected from A and S;
   X₁₀ is selected from A, S and R;
   X₁₄ is selected from A, S, C and K, preferably A, S and K;
   X₂₆ is selected from D and E;
   X₃₉ is selected from D and E;
   X₄₀ is selected from A and E;
   X₄₃ is selected from A and K;
   X₄₄ is selected from A, S and E;
   L in position 45 is present or absent; and
   P in position 46 is present or absent;
and bb) an amino acid sequence which has at least 95% identity to the sequence defined in aa), provided that X₇ is neither L, E or D.

Further, the ABR may, as in EP2590993B1 comprise an amino acid sequence selected from
aaa) LAX₃AKEAANA ELDX₁₄YGVSDF YKRLIDKAKT VEGVEALKDAILAALP, wherein, independently of each other,
   X₃ is selected from E and S;
   X₁₄ is selected from A, S, C and K, preferably A, S and K;
and bbb) an amino acid sequence which has at least 95% identity to the sequence defined in aaa).

In one embodiment, the ABR comprises an amino acid sequence selected from the group consisting of: 
LAEAKVLANR ELDKYGVSDF YKRLINKAKT VEGVEALKLH ILAALP (SEQ ID NO:1);
LAEAKEAANA ELDSYGVSDF YKRLIDKAKT VEGVEALKDA ILAALP (SEQ ID NO:2);
GLAEAKEAAN AELDSYGVSD FYKRLIDKAK TVEGVEALKD AILAALP (SEQ ID NO:3);
LAEAKVLANR ELDKYGVSDY YKNLINNAKT VEGVKALIDE ILAALP (SEQ ID NO:4); and
LAEAKVLALR ELDKYGVSDY YKDLIDKAKT VEGVKALIDE ILAALP (SEQ ID NO:5).

A particularly preferred group consists of SEQ ID NO:1 (ABD₃₅), SEQ ID NO:2 and SEQ ID NO:3 (PEP07914 in WO2012004384).

The cytotoxic radionuclide is preferably selected from the group consisting of ¹⁷⁷Lu, ⁹⁰Y, ¹⁸⁸Re; ¹⁸⁶Re; ¹⁶⁶Ho, ¹⁵³Sm, ⁶⁷Cu, ⁶⁴Cu, ¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.

A preferred radionuclide is ¹⁷⁷Lu.

⁷⁷Lu, ⁹⁰Y, ¹⁸⁸Re; ¹⁸⁶Re; ¹⁶⁶Ho, ¹⁵³Sm, ⁶⁷Cu, ⁶⁴Cu, ¹⁴⁹Tb, ¹⁶¹Tb, ⁴⁷Sc; ²²⁵Ac; ²¹²Pb; ²¹³Bi, ²¹²Bi, ²²⁷Th, ²²³Ra and ^{58m}Co are radiometals that may be bound to the fusion protein by means of chelator-based conjugation. The chelator is preferably covalently bound to a cysteine residue of the fusion protein, optionally via a thiol-reactive linker. Binding to an amine of an amino acid residue of the fusion protein is also possible, but generally less preferred.

The chelator may be selected from the group consisting of DOTA and its derivatives (e.g. the maleimido-derivative of DOTA), cross-bridged macrocyclic chelators and sterically-restricted acyclic chelators.

Particularly suitable chelators for ¹⁷⁷Lu, ⁹⁰Y, ¹⁶⁶Ho, ¹⁵³Sm, ¹⁴⁹Tb, ¹⁶¹Tb, ⁴⁷Sc; ²²⁵Ac; ²¹²Pb; ²¹³Bi, ²¹²Bi, ²²⁷Th and ^{58m}Co are DOTA and its derivative DOTAGA.

For ⁶⁷Cu and ⁶⁴Cu a cross-bridged chelator, such as CB-TE2A, is a better option.

For ¹⁸⁸Re and ¹⁸⁶Re a chelator based on a cysteine- or mercaptoacetyl-containing peptide is preferred.

¹³¹I, ⁷⁶As, ⁷⁷As and ²¹¹At are non-metal radionuclides that can be bound to the fusion protein by means of covalent conjugation.

The radioiodination may be achieved using ((4-hydroxyphenyl)ethyl) maleimide (HPEM), which can be bound to a cysteine (C) residue of the fusion protein. ⁷⁶As and ⁷⁷As (and ⁷⁴As) in As (III) form may be coupled directly to a (freshly) reduced thiol group of a cysteine (C) residue of the fusion protein.

For coupling of ²¹¹At, N-[4-(tri-n-butylstannyl)phenethyl]-maleimide can be used as a linker. In such case, ²¹¹At is first coupled to the linker by astatodestannylation forming 4-astato-phenethyl-maleimide (AtPEM), which in turn can be coupled to a cysteine (C) residue of the fusion protein.

Consequently, the cytotoxic radionuclide is preferably linked to the fusion protein via a cysteine (C) residue of the fusion protein. To avoid cross/side reactions, the fusion protein in such case preferably comprises only one cysteine (C) residue.

The cysteine (C) residue that links the cytotoxic radionuclide to the fusion protein may be a placed in a terminal position. Preferably, the cysteine (C) residue is the C-terminal residue of the fusion protein.

In one embodiment, the cysteine (C) residue is the C-terminal residue of an amino acid sequence extending from the C-terminal end of the ABR. Such an amino acid sequence may for example be GSSC.

The fusion protein may further comprise an additional region, e.g. comprising or consisting of the amino acid sequence DEAVDANS (SEQ ID NO:25) or MGDEAVDANS (SEQ ID NO:18). This additional region is typically a terminal region of the fusion protein, preferably an N-terminal region of the fusion protein. In an embodiment, the additional region extends from the N-terminal end of the HBR. The additional region may result in improved biodistribution and facilitate production.

At the same time, it is beneficial to limit the size of the therapeutic conjugate of the present disclosure. Accordingly, it preferably comprises no more than 150 amino acid residues, such as no more than 130 amino acid residues. The total molecular weight of the therapeutic conjugate is preferably below 20 kDa, such as below 15 kDa.

In an embodiment, the fusion protein comprises the amino acid sequence of SEQ ID NO:21 (ADAPT6-(SSSG)₃-ABD₃₅), SEQ ID NO:22 (ABD₃₅-(SSSG)₃-ADAPT6), SEQ ID NO:23 (ADAPT6-(SSSG)₅-ABD₃₅) or SEQ ID NO:24 (ABD₃₅-(SSSG)₅-ADAPT6). Out of these four sequences, SEQ ID NO:21 (ADAPT6-(SSSG)₃-ABD₃₅) is considered to be the most preferred.

In an embodiment, the fusion protein consists of the amino acid sequence of SEQ ID NO:27, SEQ ID NO:28, SEQ ID NO:29 or SEQ ID NO:30. Out of these four sequences, SEQ ID NO:27 is considered to be the most preferred.

The present disclosure further provides a pharmaceutical composition comprising the therapeutic conjugate discussed above and a pharmaceutically acceptable carrier.

The composition is preferably adapted for intravenous administration. Accordingly, the composition is typically water-based. The water-based composition is preferably buffered, such as phosphate-buffered. As an example, the composition may be based on phosphate-buffered saline. The water-based composition may comprise human serum albumin (HSA). HSA scavenges free radicals and prevents radiolytic damages to the therapeutic conjugate. The amount of HSA may be 10-150 mg/ml, such as 50-100 mg/ml, preferably 75 mg/ml.

In one embodiment, the composition comprises pentetic acid (DTPA). DTPA converts free nuclide into a form that is rapidly excreted via kidneys. The amount of DTPA may be 0.1-1.0 mg/ml, such as 0.2-0.8 mg/ml, preferably 0.4 mg/ml.

The present disclosure further provides the above-mentioned therapeutic conjugate for use in a method of treating a cancer, typically a cancer overexpressing HER2. Diagnosis of HER2 overexpression is known to the skilled person. In an embodiment, the cancer has developed resistance to a first line HER2-targeting treatment, such as an antibody-based treatment, e.g. trastuzumab, pertuzumab or antibody-drug conjugated trastuzumab-DMi. The cancer type is preferably breast cancer or gastric/gastroesophageal cancer.

The method may comprise intravenous administration of the therapeutic conjugate, e.g. in the pharmaceutical composition described above. The number of injections of the therapeutic conjugate maybe 1-5, such as 1-3. The injections are typically carried out several days apart. A low number of injections is preferred, not only from a cost perspective, but also to minimize the pain and possible side effects of the injections.

### EXAMPLES - MATERIALS AND METHODS

### Statistics

Statistical treatment was performed using GraphPad Prism software (version 4.00 for Microsoft Windows; GraphPad Software) to determine significant differences (P < 0.05). Data on cellular uptake and processing and biodistribution were analyzed by unpaired 2-tailed t test when 2 groups were compared. Comparison of data for more than 2 groups was performed using ANOVA with a Bonferroni test for multiple comparison. Biodistribution data concerning dual-label studies were analyzed using paired t test.

### Production, Purification, Conjugation and Characterization of Targeting Proteins

An ABD variant was selected as the ABR. This variant, referred to as ABD₃₅ (SEQ ID NO:1), is engineered to bind human serum albumin with subpicomolar affinity (Jonsson 2008). Further, ADAPT6 (SEQ ID NO:6) was selected as the HBR because it shows high affinity to HER2. SSSG repeats were selected for the spacer region.

Genes encoding ADAPT6-(SSSG)₃-ABD₃₅ (SEQ ID NO:21), ABD₃₅-(SSSG)₃-ADAPT6 (SEQ ID NO:22), ADAPT6-(SSSG)₅-ABD₃₅ (SEQ ID NO:23) and ABD₃₅-(SSSG)₅-ADAPT6 (SEQ ID NO:24) were synthesized by Thermo Fisher Scientific (Waltham, MA, USA) and subcloned into expression vectors through amplification with primers containing the desired restriction sites as well as the N-terminal sequence MGDEAVDANS (SEQ ID NO:18) and the C-terminal sequence GSSC. Proteins were expressed in *E. coli* BL21*(DE3) cells and extracted by sonication. The proteins were purified by loading the lysates on an in-house produced affinity chromatography column coupled with Human Serum Albumin (HSA).

Proteins intended for ¹⁷⁷Lu labeling were conjugated with the chelator DOTA (Macrocyclics, TX, USA) through standard maleimide coupling at the free thiol group of the C-terminal cysteine. Both the DOTA-conjugated as well as the unconjugated protein intended for ¹²⁵I labeling, were further purified through semipreparative RP-HPLC (Zorbax, 300SB-C18, 9.4 × 250 mm, 5 µm, Agilent).

Non-fused ADAPT6 (MGDEAVDANS-ADAPT6-GSSC, SEQ ID NO:26) was used as a control in the biodistribution measurements and was produced and purified like previously described (Garousi 2019).

For the purpose of radionuclide therapy, a non-target binding version of ADAPT was designed, denoted ADAPT_{Neg} (SEQ ID NO:19).

The gene of a non-target-binding fusion protein (MGDEAVDANS-ADAPT_{Neg}-(SSSG)₃-ABD₀₃₅-GSSC (SEQ ID NO:20)) was synthesized by Thermo Fisher Scientific (Waltham, MA, USA) and produced as described above.

The molecular weights of all proteins were confirmed by liquid chromatography-electro-spray ionization-mass spectrometry (LC-ESI-MS) using an Impact II UHR QqTOF MS (Bruker Daltonics, MA, USA) and the secondary structure and thermal stability was evaluated on a Chirascan circular dichroism spectrometer (Applied Photophysics, Surrey, UK) like previously described (Garousi 2019).

Target binding analysis was performed on a Biacore T200 system (GE Healthcare, Stockholm, Sweden) by injecting analytes over a CM5 chip immobilized with Murine Serum Albumin (MSA), Human Serum Albumin (HSA) and Human Epidermal growth factor receptor 2 (HER2) to a response level of ∼1000 RU each. Experimental parameters have been described previously (Garousi 2019).

### Radiolabeling

Yield of ¹²⁵I-HPEM was measured by radio-TLC using Silica gel 60 F254 TLC plates (20×100 mm, elution path 80 mm; E. Merck, Darmstadt, Germany) eluted with acetonitrile. Radiochemical yield and purities were measured using radio-ITLC (Varian Medical Systems, Palo Alto, CA, USA). For analysis of radioiodinated proteins, a mixture of acetone:water (7:3) was used for development. In analysis of ¹⁷⁷Lu-labeled proteins, 0.2 M citric acid, pH 2.0, was used for development. To cross-validate radio-ITLC data, radio-HPLC analysis was performed. Hitachi Chromaster HPLC systems with radioactivity detector and Vydac RP C18 column (300 Å; 3x150 mm; 5-µm) at room temperature was used. Solvent A was 0.1% trifluoroacetic acid (TFA) in H₂O; solvent B was 0.1% TFA in acetonitrile. The flow rate was 1 mL/min, with a 5% B to 80% B gradient over 20 min.

Labelling with ¹²⁵I was performed according to the method described by (Tolmachev 2009). Briefly, ¹²⁵I (14 MBq) was mixed with a solution of HPEM (10 µg, 31.5 nmol) in 5% acetic acid in methanol. Chloramine-T (10 µL, 8 mg/mL in MQ water) and 10 µL 5% acetic acid in methanol were added, and the mixture was incubated for 5 min at room temperature. The reaction was quenched by adding 10 µL sodium metabisulphite (12 mg/mL in water). ADAPT6 derivatives (500 µg, 38.9 nmol) were reduced by incubation in PBS with 20 mM dithiothreitol (DTT) for 60 min at 40°C. Reduced proteins were purified using size-exclusion NAP-5 column (GE Healthcare, Uppsala, Sweden) pre-equilibrated with NH₄OAc 0.2 M buffer, pH 6. Radioiodinated HPEM was added, and the mixture was incubated for 60 min at 40°C. Radiolabeled proteins were purified using NAP-5 columns.

Labeling with ¹⁷⁷Lu was performed as previously described (Altai 2017). Briefly, a DOTA-conjugated protein (80 µg, 6.2 nmol) in 1 M ascorbate buffer, pH 5.5, was mixed with 345 MBq ¹⁷⁷Lu in 0.1 M HCl. The mixture was incubated for 45 min at 95°C. Thereafter, a 5000-fold molar excess of EDTA was added to scavenge a loosely bound nuclide, and the mixture was incubated for 15 min at 95°C. Radiolabeled proteins were purified using NAP-5 columns.

Stability of radioiodine labels was tested by 3-h incubation with 1000-fold molar excess of potassium iodide. Stability of labeling with ¹⁷⁷Lu was evaluated by 3-h challenge with 5000-fold molar excess of EDTA.

### In vitro evaluation

Cell-lines were purchased from American Type Culture Collection (ATCC, Manassas, VA). The specificity of binding of the radiolabeled ADAPT6 derivatives to HER2-expressing cancer cells was evaluated using ovarian carcinoma SKOV-3 and breast carcinoma BT474 cell lines using a saturation method described earlier (Wallberg 2008). The affinity of radiolabeled conjugates binding to living HER2-expressing SKOV-3 cells was determined using LigandTracer (Ridgeview Instruments AB) by an established method as described previously (Tolmachev 2014). Internalization of radiolabeled ADAPT6 derivatives by SKOV-3 and BT474 cells during continuous incubation was evaluated by a modified acid wash method as described earlier (Wallberg 2008).

### Animal studies

Animal studies were planned in agreement with Swedish national legislation concerning protection of laboratory animals and were approved by the Ethics Committee for Animal Research in Uppsala.

Biodistribution and targeting properties were evaluated in BALB/C nu/nu mice bearing HER2-positive SKOV-3 xenografts. As a specificity control, HER2-negative Ramos xenografts were used. To establish xenografts, 107 cells were implanted subcutaneously.

### Biodistribution measurements

To reduce the number of tumor-bearing mice, a dual-label approach was used. The mice were injected into tail vein with a mixture of ¹²⁵I- and ¹⁷⁷Lu-labeled ADAPT6 variants (15 kBq of ¹²⁵I and 90 kBq of ¹⁷⁷Lu per animal in 100 mL of PBS). The total injected protein dose was adjusted to 3 µg/mouse for un-fused ADAPT6 and 6 µg/mouse for ABD-fused variants using the corresponding unlabeled protein. After exsanguination under anesthesia, the organ and tissues of interest were excised and their activity was measured using an automated gamma-spectrometer (1480 WIZARD; Wallac Oy). Activity was measured in the energy window from 10 to 45 keV for ¹²⁵I, and from 90 to 370 keV for ¹⁷⁷Lu.

To evaluate influence of fusion with ABD₀₃₅, effect of positioning of ABD₀₃₅ relative to ADAPT6 moiety and a chemical nature of a label, biodistribution of MG-DEAVDANS-ADAPT6-(SSSG)₃-ABD₀₃₅-GSSC-HPEM-¹²⁵I, MG-DEAVDANS-ADAPT6-(SSSG)₃-ABD₀₃₅-GSSC-DOTA-¹⁷⁷Lu, MG-DEAVDANS-ABD₀₃₅-(SSSG)₃-ADAPT6-GSSC-HPEM-¹²⁵I, MG-DEAVDANS-ABD₀₃₅-(SSSG)₃-ADAPT6-GSSC-DOTA-¹⁷⁷Lu, MG-DEAVDANS-ADAPT6-GSSC-HPEM-¹²⁵I and MG-DEAVDANS-ADAPT6-GSSC-DOTA-¹⁷⁷Lu was compared 48 h after injection. Five mice per group was used in this experiment. The average mouse weight was 16.9 ± 0.6 g, and the average tumor weight 0.42 ± 0.6 g.

To evaluate dosimetry of the most promising variants, MG-DEAVDANS-ADAPT6-(SSSG)₃-ABD₀₃₅-GSSC-HPEM-¹²⁵I and MG-DEAVDANS-ADAPT6-(SSSG)₃-ABD₀₃₅-GSSC-DOTA-¹⁷⁷Lu, their biodistribution was measured 4, 24, 48, 72, 168 and 336 h after injection in mice bearing SKOV-3 xenografts (average mouse weight was 16.3 ± 1.3 g, and average tumor weight 0.11 ± 0.06 g). To control that the tumor uptake is HER2-specific, the biodistribution of these conjugates in mice bearing HER2-negative Ramos xenografts (average tumor weight 0.11 ± 0.06 g) was measured 48 h after injection. Four mice per data point were used in this experiment. Dosimetry was evaluated as described earlier (Westerlund 2019).

### Radionuclide therapy

To evaluate the therapeutic effect of the ADAPT6 fused to ABD, 107 SKOV-3 cells per mouse were implanted subcutaneously in the abdomen. At least twice a week, the mice were weighed and visually inspected, and tumors were measured using electronic calipers. Tumor volumes (mm³) were calculated as [length (mm)] · [width (mm)]²·0.5. The animals were euthanized when tumors reached a size of 1,000 mm³ or became ulcerated, or if an animal's weight dropped by more than 10% during 1 week or by more than 15% since the study began. After euthanasia, tumors and kidneys were excised for subsequent histologic evaluation.

Treatment started 7 d after tumor implantation, when the average tumor volume was 0.07 ± 0.02 mm³ and the average mouse weight was 15.1 ± 0.4 g. The mice were randomly divided into 5 groups of 11 animals each. The first group of treated animals was injected with 6 µg (4.7 nmol)/18 MBq of MG-DEAVDANS-ADAPT6-(SSSG)₃-ABD₀₃₅-GSSC-DOTA-¹⁷⁷Lu. Animals in the second treatment group received two treatments, the first at day 7 and the second three weeks after the first one. Control group 1 received vehicle alone, PBS. To evaluate the influence of non-labeled ABD-fused ADAPT6 on tumor growth, control group 2 was injected with 6 µg (4.7 nmol) of unlabeled MG-DEAVDANS-ADAPT6-(SSSG)₃-ABD₀₃₅-GSSC-DOTA. To evaluate the effect of nontargeting radiolabeled protein, a control group 3 was injected with MG-DEAVDANS-ADAPT_{Neg}-(SSSG)₃-ABD₀₃₅-GSSC-DOTA-¹⁷⁷Lu (6 µg/18 MBq) only.

To confirm tumor targeting, SPECT/CT imaging was performed. A mouse was imaged at 72 h as described in (Westerlund 2018).

### EXAMPLES - RESULTS

### Production, Purification, Conjugation and Characterization of Targeting Proteins

All proteins used in this study was produced in E. coli and purified to homogeneity. To establish the optimal length of the SSSG repeat linker between the HBR and the ABR, an SPR binding analysis was performed to compare affinities between variants with the (SSSG)₃ spacer region and the longer (SSSG)₅ spacer region. As can be seen in Table 1, the SPR data show no gain of affinity when the length of the spacer region was increased from 12 to 20 amino acid residues. Therefore, the rest of the study was focused on the shorter variants.

**Table 1. Affinity constants for the ABD-fused proteins towards HSA, MSA and HER2. All variants demonstrate similar affinities regardless of the placement of the ABD and the length of the linker.**

| | SEQ ID NO:# | K_{D} (MSA), M | K_{D} (HSA), M | K_{D} (HER2), M |
|---|---|---|---|---|
| MG-DEAVDANS-ADAPT6-(SSSG)₃-ABD₃₅-GSSC | 27 | 1.76 × 10⁻⁹ | 4.98× 10⁻¹¹ | 4.67 × 10⁻⁹ |
| MG-DEAVDANS-ABD₃₅-(SSSG)₃-ADAPT6-GSSC | 28 | 1.83 × 10⁻⁹ | 5.21× 10⁻¹¹ | 5.20 × 10⁻⁹ |
| MG-DEAVDANS-ADAPT6-(SSSG)₅-ABD₃₅-GSSC | 29 | 1.80 × 10¹¹ | 5.38 × 10⁻¹¹ | 4.45 × 10⁻⁹ |
| MG-DEAVDANS-ABD₃₅-(SSSG)₅-ADAPT6-GSSC | 30 | 1.33 × 10⁻¹¹ | 5.20 × 10⁻¹¹ | 4.32 × 10⁻⁹ |

The purity of the variants intended for *in vivo* studies were determined by RP-HPLC to be above 95% (Figure 2) and mass spectrometry confirmed the correct mass of all proteins (Table 2). Circular dichroism analysis indicated high alpha helical content as well as ability to completely refold after heat-treatment of the sample (Figure 3). All variants demonstrated high melting temperatures, almost identical to the non-fused control (MGDEAVDANS-ADAPT6-GSSC).

**Table 2. Theoretical and experimental molecular weights of fusion proteins used in this study.**

| | Without DOTA | | With DOTA | |
|---|---|---|---|---|
| | Theoretical MW (Da) | Measured MW (Da) | Theoretical MW (Da) | Measured MW (Da) |
| MG-DEAVDANS-ADAPT6-(SSSG)₃-ABD₃₅-GSSC (SEQ ID NO:27) | 12338.96 | 12338.45 | 12865.50 | 12864.69 |
| MG-DEAVDANS-ABD₃₅-(SSSG)₃-ADAPT6-GSSC (SEQ ID NO:28) | 12338.96 | 12338.46 | 12865.50 | 12864.69 |
| MG-DEAVDANS-ADAPT6-GSSC (SEQ ID NO:26) | 6292.10 | 6291.21 | 6818.64 | 6817.47 |
| MG-DEAVDANS-ADAPT_{Neg}-(SSSG)₃-ABD₃₅-GSSC (SEQ ID NO:20) | - | - | 12749.33 | 12748.62 |

SPR measurements of the ABD-fused constructs revealed similar affinities towards HER2 (around 5 nM), regardless of the placement of the ABD. This is comparable to the previously determined 4 nM affinity of the unfused control (Garousi 2016). The affinity towards the serum albumins were also similar between all constructs, with measured affinities around 1.8 nM towards MSA and around 50 pM for HSA (Figure 4, Table 1). Figure 4 also demonstrates simultaneous binding of HER2 and HSA, since the fusion proteins can bind HER2 also in the presence of saturating concentrations of HSA. Further strengthening this claim, it can be seen that the saturated proteins no longer bind HSA/MSA.

As previously mentioned, a non-target binding fusion protein was designed to be used as a control in the therapy studies. SPR measurements confirm that the three-point mutations efficiently remove the binding to HER2 (Figure 5).

### Radiolabeling

Data concerning radiochemical yields and radiochemical purity are provided in Table 3. All labeling protocols were efficient and purification using NAP-5 provided complete absence of non-conjugated radionuclide. Radiochemical yield of labelling of HPEM with ¹²⁵I (covalent coupling) was 98%. For experimental therapy, the maximum molar activity up to 55.6 GBq/µmol was obtained for MG-DEAVDANS-ADAPT6-(SSSG)₃-ABD₀₃₅-GSSC-DOTA-¹⁷⁷Lu. Identity of radiolabeled ADAPT6 derivative was confirmed by radio-HPLC (Figures 6 and 7). All labels were stable under challenge conditions (no measurable radionuclide release).

**Table 3. Radiochemical yields and purity of radiolabeled ADAPT6 variants.**

| | Radiochemical yield (%) | Radiochemical purity (%) |
|---|---|---|
| MG-DEAVDANS-ADAPT6-GSSC-HPEM-¹²⁵I | 63±11 | 100 |
| MG-DEAVDANS-ABD₀₃₅-(SSSG)₃-ADAPT6-GSSC-HPEM-¹²⁵I | 65±25 | 100 |
| MG-DEAVDANS-ADAPT6-(SSSG)₃-ABD₀₃₅-GSSC-HPEM-¹²⁵I | 68±18 | 100 |
| MG-DEAVDANS-ADAPT6-GSSC-DOTA-¹⁷⁷Lu | 96±1 | 100 |
| MG-DEAVDANS-ABD₀₃₅-(SSSG)₃-ADAPT6-GSSC-DOTA-¹⁷⁷Lu | 94±0.1 | 100 |
| MG-DEAVDANS-ADAPT6-(SSSG)₃-ABD₀₃₅-GSSC-DOTA-¹⁷⁷Lu | 94.1±0.1 | 100 |

### In vitro evaluation

Pre-saturation of HER2 receptors in cells with unlabeled constructs resulted in a significant (p < 5 × 10⁻⁵) reduction of binding of all radiolabeled ADAPT6 variants to both SKOV-3 and BT-474 cell lines. Data concerning binding and processing of radiolabeled ADAPT6 derivatives by HER2-expressing cells are presented in Figure 8 and 9. The binding pattern of radiometal-labeled ABD₀₃₅-fused variant was similar to the pattern for the non-fused variant; rapid binding during first two hours followed by somewhat slower increase. The data for non-fused variant closely resembled the data for the ¹¹¹In-labeled counterpart (Lindbo 2018). The internalization rate for all variants was slow and less than 20% of the cell-bound activity was internalized by 24 h.

Data concerning affinity of radiolabeled ADAPT6 derivatives to living HER2 expressing cells are presented in Table 4 and Figure 10. All variants demonstrated two interactions with HER2 on living cells, a strong one with subnanomolar apparent dissociation constant and a weaker one with a dissociation constant with the range of 20-50 nM. There was no obvious negative effect of ABD₀₃₅ fusion on the strength of ADAPT6 binding to HER2-expressing cells.

**Table 4. K_{D} for Interaction of Radiolabeled ADAPT6 Variants with HER2-Expressing SKOV-3 Cells, Determined Using LigandTracer.**

| | K_{D}1 (pM) | K_{D}2 (nM) |
|---|---|---|
| MG-DEAVDANS-ADAPT6-GSSC-HPEM-¹²⁵I | 410 ± 175 | 27 ± 7 |
| MG-DEAVDANS-ABD₀₃₅-(SSSG)₃-ADAPT6-GSSC-HPEM-¹²⁵I | 150 ± 25 | 28 ± 2 |
| MG-DEAVDANS-ADAPT6-(SSSG)₃-ABD₀₃₅-GSSC-HPEM-¹²⁵I | 600 ± 140 | 36 ± 9 |
| MG-DEAVDANS-ADAPT6-GSSC-DOTA-¹⁷⁷Lu | 337 ± 150 | 41 ± 9 |
| MG-DEAVDANS-ABD₀₃₅-(SSSG)₃-ADAPT6-GSSC-DOTA-¹⁷⁷Lu | 432 ± 158 | 38 ± 1 |
| MG-DEAVDANS-ADAPT6-(SSSG)₃-ABD₀₃₅-GSSC-DOTA-¹⁷⁷Lu | 485 ± 88 | 33 ± 4 |

### Biodistribution measurements

Effect of fusion with ABD₀₃₅ is demonstrated in Figure 11 using conjugates with a residualizing ¹⁷⁷Lu label. The biodistribution of MG-DEAVDANS-ADAPT6-GSSC-DOTA-¹⁷⁷Lu was typical for radiometal-labeled derivatives of this protein (Lindbo 2018), i.e. a reasonably high tumor uptake, rapid clearance from blood and other tissues, and very high reabsorption and retention of activity in kidneys. Fusion with ABD changes the biodistribution pattern dramatically. The retention in blood increased more than 200-fold for both variants. The renal accumulation of activity was reduced 10-fold for MG-DEAVDANS-ABD₃₅-(SSSG)₃-ADAPT6-GSSC-DOTA-¹⁷⁷Lu and 14-fold for MG-DEAVDANS-ADAPT6-(SSSG)₃-ABD₃₅-GSSC-DOTA-¹⁷⁷Lu. At the same time, the tumor uptake of ABD-fused ADAPT6 was more than 3-fold higher than for MG-DEAVDANS-ADAPT6-GSSC-DOTA-¹⁷⁷Lu. In both cases, the tumor uptake was higher than the renal uptake.

Effect on biodistribution, caused by the positioning of ABD₀₃₅ region relative to the ADAPT6 region as well as by the chemical nature of the label, is shown in Figure 12. For the ¹²⁵I-labelled fusion proteins, the tumor uptake is relatively high (about 15%-20%), especially when compared to the kidney uptake. In case of ¹²⁵I-labeling, there is also a slight trend towards higher tumor uptake for the ADAPT6-ABD₃₅-based fusion protein than for the ABD₃₅-ADAPT6-based fusion protein. For the ¹⁷⁷Lu-labelled fusion proteins, the tumor uptake is even higher (above 25%). The highest tumor uptake (about 30%) is observed for the ¹⁷⁷Lu-labelled ADAPT6-ABD35-based fusion protein. For the ¹⁷⁷Lu-labelled fusion proteins, the kidney uptake is higher than for the ¹²⁵I-labelled fusion proteins, but still much lower than for the reference product lacking ABD₃₅. Placement of ABD₃₅ at the C-terminal end of ADAPT6 resulted in a more favorable tumor-to-kidney ratio in case of the ¹⁷⁷Lu label.

Data concerning biodistribution of MG-DEAVDANS-ADAPT6-(SSSG)₃-ABD₀₃₅-GSSC-HPEM-¹²⁵I and MG-DEAVDANS-ADAPT6-(SSSG)₃-ABD₀₃₅-GSSC-DOTA-¹⁷⁷Lu up to 48 hours after injection are presented in Figure 13 and Tables 5 and 6. In vivo specificity test has demonstrated that uptake of both variants is significantly (p < 0.005) higher in HER2-positive than in HER2-negative xenografts 48 h after injection (Figure 13). The difference was 14-17-fold.

The biodistribution data (Tables 5 and 6) demonstrate slow clearance of activity from blood. The biological half-lives in blood were 29.4 (95% CI 25.7 to 33.7) and 28.4 (95% CI 24.6 to 32.8) h for MG-DEAVDANS-ADAPT6-(SSSG)₃-ABD₀₃₅-GSSC-HPEM-¹²⁵I and MG-DEAVDANS-ADAPT6-(SSSG)₃-ABD₀₃₅-GSSC-DOTA-¹⁷⁷Lu, respectively. The level of the blood-borne activity was slightly but significantly (p < 0.05 in a paired t-test) higher for the radioiodine label at all time points. Both conjugates showed efficient targeting, as already by 24 h, the uptake in HER2-positive tumors exceeded uptake in any other organ or tissues for both variants. Starting from 72 h after injection, the tumor uptake was higher for ¹⁷⁷Lu label. Overall, the use of ¹⁷⁷Lu provided significantly higher tumor-to-blood but lower tumor-to-kidney ratios compared to ¹²⁵I label.

**Table 5. Biodistribution of MG-DEAVDANS-ADAPT6-(SSSG)₃-ABD₀₃₅-GSSC-DOTA-¹⁷⁷Lu in BALB/C nu/nu mice bearing SKOV-3 xenografts. Data are expressed as %ID/g and are averages from 4 animals ± SD.**

| | 4 h | 24 h | 48 h | 72 h | 168 h | 336 h |
|---|---|---|---|---|---|---|
| Blood | 27 ± 2 | 17 ± 2 | 11.9 ± 0.4 | 8.2 ± 1.4 | 2.5 ± 0.5 | 0.29 ± 0.08 |
| Heart | 8 ± 2 | 6 ± 1 | 5.0 ± 0.7 | 4.2 ± 0.7 | 1.8 ± 0.4 | 0.97 ± 0.07 |
| Lung | 12 ± 4 | 8 ± 1 | 6 ± 2 | 5.3 ± 0.7 | 2.3 ± 0.6 | 0.82 ± 0.07 |
| Salivary Gland | 4.8 ± 0.5 | 4.8 ± 0.6 | 4.3 ± 0.3 | 4.9 ± 0.5 | 2.6± 0.4 | 1.2 ± 0.2 |
| Liver | 6.0 ± 0.7 | 4.8 ± 0.3 | 5.2 ± 0.3 | 6.2 ± 0.6 | 3.5 ± 0.8 | 1.3 ± 0.2 |
| Spleen | 4.7 ± 0.8 | 6.2 ± 0.4 | 6.8 ± 1.0 | 6.6 ± 0.3 | 7 ± 3 | 3.8 ± 0.5 |
| Pancreas | 2.6 ± 0.5 | 1.8 ± 0.2 | 1.9 ± 0.1 | 1.6 ± 0.5 | 0.8 ± 0.3 | 0.5 2± 0.07 |
| Stomach | 2.3 ± 0.3 | 1.8 ± 0.3 | 1.4 ± 0.2 | 1.3 ± 0.1 | 0.6 ± 0.2 | 0.4 ± 0.1 |
| Small Intestines | 2.8 ± 0.6 | 2.5 ± 0.5 | 1.9 ± 0.2 | 1.7 ± 0.1 | 0.7 ± 0.3 | 0.35 ± 0.06 |
| Large Intestines | 2.4 ± 0.2 | 2.1 ± 0.2 | 1.5 ± 0.5 | 1.6 ± 0.3 | 0.8 ± 0.4 | 0.5 ± 0.1 |
| Kidney | 10.8 ± 1.3 | 10.9 ± 0.7 | 9.6 ± 0.7 | 9.3 ± 0.7 | 5 ± 1 | 1.6 ± 0.2 |
| Tumor | 9 ± 2 | 26 ± 4 | 35 ± 12 | 30 ± 5 | 25 ± 13 | 5 ± 1 |
| Skin | 5 ± 2 | 6.1 ± 0.9 | 6.2 ± 0.9 | 7.0 ± 0.3 | 4.4 ± 0.5 | 1.3 ± 0.4 |
| Muscle | 1.5 ± 0.2 | 1.58 ± 0.09 | 1.3 ± 0.2 | 1.2 ± 0.6 | 0.57 ± 0.09 | 0.31 ± 0.08 |
| Bone | 2.2 ± 0.2 | 2.3 ± 0.2 | 3.02 ± 1.55 | 1.8 ± 0.1 | 1.1 ± 0.1 | 0.7 ± 0.1 |
| Brain | 0.5 ± 0.1 | 0.5 ± 0.2 | 0.5 ± 0.1 | 0.3 ± 0.1 | 0.10 ± 0.01 | 0.13 ± 0.02 |

**Table 6. Biodistribution of MG-DEAVDANS-ADAPT6-(SSSG)₃-ABD₀₃₅-GSSC-HPEM-¹²⁵I in BALB/C nu/nu mice bearing SKOV-3 xenografts. Data are expressed as %ID/g and are averages from 4 animals ± SD.**

| | 4 h | 24 h | 48 h | 72 h | 168 h | 136 h |
|---|---|---|---|---|---|---|
| Blood | 31 ± 2 | 19 ± 2 | 13.5 ± 0.5 | 9 ± 2 | 2.8 ± 0.6 | 0.45± 0.09 |
| Heart | 9 ± 2 | 5 ± 1 | 4.0 ± 0.7 | 3.1 ± 0.6 | 0.7 ± 0.3 | 0.13 ± 0.05 |
| Lung | 14 ± 4 | 8 ± 2 | 5.4 ± 0.9 | 4.7 ± 0.7 | 1.3 ± 0.4 | 0.18 ± 0.04 |
| Salivary Gland | 5.5 ± 0.7 | 4.2 ± 0.6 | 2.8 ± 0.2 | 2.4 ± 0.3 | 0.6 ± 0.2 | 0.09 ± 0.01 |
| Liver | 6.1 ± 0.8 | 3.2 ± 0.3 | 2.6 ± 0.2 | 2.4 ± 0.3 | 0.6 ± 0.1 | 0.11 ± 0.02 |
| Spleen | 4.3 ± 1.0 | 2.7 ± 0.2 | 2.1 ± 0.3 | 1.7 ± 0.4 | 0.8 ± 0.3 | 0.22 ± 0.06 |
| Pancreas | 3.0 ± 0.6 | 1.9 ± 0.3 | 1.5 ± 0.2 | 1.1 ± 0.3 | 0.28 ± 0.06 | 0.05 ± 0.02 |
| Stomach | 2.7 ± 0.3 | 1.8 ± 0.1 | 1.2 ± 0.1 | 1.1 ± 0.2 | 0.28 ± 0.06 | 0.05 ± 0.01 |
| Small Intestines | 3.3 ± 0.5 | 2.3 ± 0.3 | 1.50 ± 0.06 | 1.2 ± 0.1 | 0.3 ± 0.1 | 0.04 ± 0.01 |
| Large Intestines | 2.9 ± 0.2 | 2.2 ± 0.2 | 1.3 ± 0.3 | 1.3 ± 0.1 | 0.3 ± 0.1 | 0.05 ± 0.01 |
| Kidney | 8 ± 1 | 5.5 ± 0.3 | 3.8 ± 0.3 | 3.1 ± 0.4 | 0.8 ± 0.2 | 0.14 ± 0.02 |
| Tumor | 10 ± 2 | 22 ± 2 | 20 ± 2 | 17 ± 3 | 8 ± 5 | 0.6 ± 0.3 |
| Skin | 5 ± 2 | 5.1 ± 0.6 | 3.4 ± 0.1 | 2.74 ± 0.09 | 1.1 ± 0.4 | 0.10 ± 0.02 |
| Muscle | 1.8 ± 0.2 | 1.57 ± 0.07 | 1.1 ± 0.1 | 0.8 ± 0.2 | 0.24 ± 0.03 | 0.03 ± 0.02 |
| Bone | 2.6 ± 0.4 | 2.0 ± 0.2 | 1.5 ± 0.3 | 1.0 ± 0.1 | 0.4 ± 0.1 | 0.05 ± 0.03 |
| Brain | 0.5 ± 0.1 | 0.5 ± 0.2 | 0.5 ± 0.1 | 0.29 ± 0.07 | 0.08 ± 0.01 | 0.02 0.01 |

The biodistribution data of Tables 5 and 6 can be compared to that presented in the prior art document Liu 2019, in which a cytotoxic polypeptide (PE38X8 or PE25) is coupled to the C terminal end of an ADAPT6-ABD₀₃₅ fusion protein (see Tables 7 and 8). Such a comparison shows that the conjugates of the present disclosure result in a drastically improved biodistribution. Notably, the fusion toxins of Liu 2019 accumulate in the liver and/or the kidney, which means that a large proportion of the drug fails to target the tumor and that severe and probably unacceptable side effects are expected in the liver and/or the kidney. At 24 h post-injection, the blood to kidney ratio is very low (0.04 and 0.12, respectively) in Liu 2019, but much higher (1.6 and 3.5, respectively) for the inventive conjugates having cytotoxic radionuclides. Similarly, the blood to liver ratio is very low (0.3 and 0.04, respectively) in Liu 2019, but much higher for the inventive conjugates having cytotoxic radionuclides (3.5 and 5.9, respectively) at 24 h post-injection. Notably, at this point in time, Liu 2019 does not show a blood concentration above 1.8% despite having the albumin-binding region ABD₀₃₅. In contrast, the blood concentration of the conjugates of the present disclosure is 17% and 19%, respectively.

**Table 7. Biodistribution at 4 h post-injection of MG-DEAVDANS-ADAPT6-(SSSG)₃-ABD₀₃₅-GSSC-DOTA-¹⁷⁷Lu ("Inventive 1") and MG-DEAVDANS-ADAPT6-(SSSG)₃-ABD₀₃₅-GSSC-HPEM-¹²⁵I ("Inventive 2") compared to the biodistribution of ADAPT6-ABD₀₃₅-PE38X8 ("Liu 1") and ADAPT6-ABD₀₃₅-PE25 ("Liu 2") presented in Liu 2019. "B/K ratio" means blood to kidney ratio. "B/L ratio" means blood to liver ratio.**

| | Inventive 1 | Inventive 2 | Liu 1 | Liu 2 |
|---|---|---|---|---|
| Blood | 27 ± 2 | 31 ± 2 | 9.0±0.7 | 3.2±0.1 |
| Heart | 8 ± 2 | 9 ± 2 | 3.0±0.8 | 1.1±0.2 |
| Lung | 12 ± 4 | 14 ± 4 | 3.3±0.4 | 1.3±0.1 |
| Salivary Gland | 4.8 ± 0.5 | 5.5 ± 0.7 | 1.5±0.2 | 0.7±0.2 |
| Liver | 6.0 ± 0.7 | 6.1 ± 0.8 | 9.0±0.4 | 43.0±7.0 |
| Spleen | 4.7 ± 0.8 | 4.3 ± 1.0 | 3.3±0.3 | 19.0±4.0 |
| Pancreas | 2.6 ± 0.5 | 3.0 ± 0.6 | 0.9±0.2 | 0.4±0.1 |
| Stomach | 2.3 ± 0.3 | 2.7 ± 0.3 | 1.0±0.1 | 0.5±0.2 |
| Small Intestines | 2.8 ± 0.6 | 3.3 ± 0.5 | N/A | N/A |
| Large Intestines | 2.4 ± 0.2 | 2.9 ± 0.2 | N/A | N/A |
| Kidney | 10.8 ± 1.3 | 8 ± 1 | 58.0±3.0 | 13.0±1.0 |
| Tumor | 9 ± 2 | 10 ± 2 | N/A | N/A |
| Skin | 5 ± 2 | 5 ± 2 | 1.8±0.3 | 0.6±0.1 |
| Muscle | 1.5 ± 0.2 | 1.8 ± 0.2 | 0.7±0.1 | 0.3±0.1 |
| Bone | 2.2 ± 0.2 | 2.6 ± 0.4 | 1.3±0.1 | 1.1±0.3 |
| Brain | 0.5 ± 0.1 | 0.5 ± 0.1 | N/A | N/A |
| B/K ratio | 2.5 | 3.9 | 0.16 | 0.25 |
| B/L ratio | 4.5 | 5.1 | 1 | 0.07 |

**Table 8. Biodistribution at 24 h post-injection of MG-DEAVDANS-ADAPT6-(SSSG)₃-ABD₀₃₅-GSSC-DOTA-¹⁷⁷Lu ("Inventive 1") and MG-DEAVDANS-ADAPT6-(SSSG)₃-ABD₀₃₅-GSSC-HPEM-¹²⁵I ("Inventive 2") compared to the biodistribution of ADAPT6-ABD₀₃₅-PE38X8 ("Liu 1") and ADAPT6-ABD₀₃₅-PE25 ("Liu 2") presented in Liu 2019. "B/K ratio" means blood to kidney ratio. "B/L ratio" means blood to liver ratio.**

| | Inventive 1 | Inventive 2 | Liu 1 | Liu 2 |
|---|---|---|---|---|
| Blood | 17 ± 2 | 19 ± 2 | 1.8±0.1 | 1.3±0.2 |
| Heart | 6 ± 1 | 5 ± 1 | 1.0±0.1 | 0.7±0.2 |
| Lung | 8 ± 1 | 8 ± 2 | 1.0±0.2 | 0.8±0.1 |
| Salivary Gland | 4.8 ± 0.6 | 4.2 ± 0.6 | 0.7±0.1 | 0.6±0.1 |
| Liver | 4.8 ± 0.3 | 3.2 ± 0.3 | 6.0±1.0 | 32.0±5.0 |
| Spleen | 6.2 ± 0.4 | 2.7 ± 0.2 | 2.2±0.4 | 11.4±1.3 |
| Pancreas | 1.8 ± 0.2 | 1.9 ± 0.3 | 0.4±0.1 | 0.4±0.2 |
| Stomach | 1.8 ± 0.3 | 1.8 ± 0.1 | 0.5±0.1 | 0.4±0.2 |
| Small Intestines | 2.5 ± 0.5 | 2.3 ± 0.3 | N/A | N/A |
| Large Intestines | 2.1 ± 0.2 | 2.2 ± 0.2 | N/A | N/A |
| Kidney | 10.9 ± 0.7 | 5.5 ± 0.3 | 46.0±2.0 | 11.0±1.0 |
| Tumor | 26 ± 4 | 22 ± 2 | N/A | N/A |
| Skin | 6.1 ± 0.9 | 5.1 ± 0.6 | 1.3±0.3 | 0.7±0.2 |
| Muscle | 1.58 ± 0.09 | 1.57 ± 0.07 | 0.4±0.1 | 0.3±0.1 |
| Bone | 2.3 ± 0.2 | 2.0 ± 0.2 | 0.7±0.2 | 0.8±0.2 |
| Brain | 0.5 ± 0.2 | 0.5 ± 0.2 | N/A | N/A |
| B/K ratio | 1.6 | 3.5 | 0.04 | 0.12 |
| B/L ratio | 3.5 | 5.9 | 0.3 | 0.04 |

### Radionuclide therapy

Imaging performed during experimental therapy, 72 hours post injection (Figure 14) confirmed the biodistribution data showing that ¹⁷⁷Lu was accumulated in tumors to a much higher extent than in any of the normal tissues.

The tumor growth data (Figure 15) reflect impact of treatment. The tumor volume doubling times were 7.0 (95% CI 6.0 to 8.3), 8.0 (95% CI 7.0 to 9.1), and 10.5 (95% CI 7.6 to 15.3) d, for groups of mice treated with PBS, unlabeled MG-DEAVDANS-ADAPT6-(SSSG)₃-ABD₀₃₅-GSSC-DOTA, and MG-DEAVDANS-ADAPT_{Neg}-(SSSG)₃-ABD₀₃₅-GSSC-DOTA-¹⁷⁷Lu, respectively. In mice treated with MG-DEAVDANS-ADAPT6-(SSSG)₃-ABD₀₃₅-GSSC-DOTA-¹⁷⁷Lu, an initial tumor growth was followed by a shrinkage (at 11 d), and the tumor re-growth started only at approximately 30 d for the group treated by a single injection of MG-DEAVDANS-ADAPT6-(SSSG)₃-ABD₀₃₅-GSSC-DOTA-¹⁷⁷Lu. Such tumor growth pattern had an apparent influence on survival (Figure 16). Median survival in the control groups was 25, 25, and 31 d for treatment with PBS, unlabeled MG-DEAVDANS-ADAPT6-(SSSG)₃-ABD₀₃₅-GSSC-DOTA, and MG-DEAVDANS-ADAPT_{Neg}-(SSSG)₃-ABD₀₃₅-GSSC-DOTA-¹⁷⁷Lu, respectively. Treatment with MG-DEAVDANS-ADAPT_{Neg}-(SSSG)₃-ABD₀₃₅-GSSC-DOTA-¹⁷⁷Lu slightly but significantly increases survival compared with survival of mice treated by "cold" substances. Median survival in the group receiving a single injection of MG-DEAVDANS-ADAPT6-(SSSG)₃-ABD₀₃₅-GSSC-DOTA-¹⁷⁷Lu was 70 days, which is significantly (p <0.0001) longer than the median survival of mice in the group treated with MG-DEAVDANS-ADAPT_{Neg}-(SSSG)₃-ABD₀₃₅-GSSC-DOTA-¹⁷⁷Lu. In this group, an animal with a 152-mm³ tumor at the start of therapy showed nearly complete tumor disappearance at day 62, which lasted until the end of the study (day 90). In the group treated with two injections of MG-DEAVDANS-ADAPT6-(SSSG)₃-ABD₀₃₅-GSSC-DOTA-¹⁷⁷Lu, (only) one mouse had to be sacrificed (at day 29 due to loss of body weight). Other animals had noticeable weight reduction 11-14 days after first treatment. By the day 90, one tumor demonstrated re-growth (with the doubling time of 16 days) but the tumor volumes were however below 120 mm³ in all other animals in this group.

The therapy was well tolerated. The appearance of the skin, fat pads, and eyes did not differ between treated and untreated mice, and there was no behavior indicating pain or suffering. The average animal weight did not differ significantly between the treated groups and the control groups (data not shown). The average weight in the groups treated with MG-DEAVDANS-ADAPT6-(SSSG)₃-ABD₀₃₅-GSSC-DOTA-¹⁷⁷Lu increased between day 28 and day 90, which reflects recovery from the disease and treatment (data not shown).

### REFERENCES

Altai M, Westerlund K, Velletta J, Mitran B, Honarvar H, Karlström AE. Evaluation of affibody molecule-based PNA-mediated radionuclide pretargeting: Development of an optimized conjugation protocol and 177Lu labeling. Nucl Med Biol. 2017 Nov;54:1-9.
Garousi J, Lindbo S, Nilvebrant J, Åstrand M, Buijs J, Sandström M, Honarvar H, Orlova A, Tolmachev V, Hober S. ADAPT, a Novel Scaffold Protein-Based Probe for Radionuclide Imaging of Molecular Targets That Are Expressed in Disseminated Cancers. Cancer Res. 2015 Oct 15;75(20):4364-71.
Garousi J, Lindbo S, Honarvar H, Velletta J, Mitran B, Altai M, Orlova A, Tolmachev V, Hober S. Influence of the N-Terminal Composition on Targeting Properties of Radiometal-Labeled Anti-HER2 Scaffold Protein ADAPT6. Bioconjug Chem. 2016 Nov 16;27(11):2678-2688.
Garousi J, Lindbo S, Borin J, von Witting E, Vorobyeva A, Oroujeni M, Mitran B, Orlova A, Buijs J, Tolmachev V, Hober S. Comparative evaluation of dimeric and monomeric forms of ADAPT scaffold protein for targeting of HER2-expressing tumours. Eur J Pharm Biopharm. 2019 Jan;134:37-48.
Jonsson A, Dogan J, Herne N, Abrahmsén L, Nygren PA. Engineering of a femtomolar affinity binding protein to human serum albumin. Protein Eng Des Sel. 2008 Aug;21(8):515-27.
Krasniqi A, D'Huyvetter M, Devoogdt N, Frejd FY, Sörensen J, Orlova A, Keyaerts M, Tolmachev V. Same-Day Imaging Using Small Proteins: Clinical Experience and Translational Prospects in Oncology. J Nucl Med. 2018 Jun;59(6):885-891.
Lindbo S, Garousi J, Åstrand M, et al. Influence of histidine-containing tags on the biodistribution of ADAPT scaffold proteins. Bioconjug Chem. 2016;27:716-726.
Lindbo S, Garousi J, Mitran B, Altai M, Buijs J, Orlova A, Hober S, Tolmachev V. Radionuclide Tumor Targeting Using ADAPT Scaffold Proteins: Aspects of Label Positioning and Residualizing Properties of the Label. J Nucl Med. 2018 Jan;59(1):93-99.
Nilvebrant J, Hober S. The albumin-binding domain as a scaffold for protein engineering. Comput Struct Biotechnol J. 2013;6:1-8.
Nilvebrant J, Hober S. Engineering of Bispecific Affinity Proteins with High Affinity for ERBB2 and Adaptable Binding to Albumin. PLOS ONE. August 2014, Volume 9, Issue 8.
Price EW, Orvig C. Matching chelators to radiometals for radiopharmaceuticals. Chem Soc Rev. 2014 Jan 7;43(1):260-90.
Tolmachev V, Mume E, Sjöberg S, Frejd FY, Orlova A. Influence of valency and labelling chemistry on in vivo targeting using radioiodinated HER2-binding Affibody molecules. Eur J Nucl Med Mol Imaging. 2009;36:692-701.
Tolmachev V, Orlova A, Andersson K. Methods for radiolabelling of monoclonal antibodies. Methods Mol Biol. 2014;1060:309-30.
Westerlund K, Altai M, Mitran B, Konijnenberg M, Oroujeni M, Atterby C, de Jong M, Orlova A, Mattsson J, Micke P, Karlström AE, Tolmachev V. Radionuclide Therapy of HER2-Expressing Human Xenografts Using Affibody-Based Peptide Nucleic Acid-Mediated Pretargeting: In Vivo Proof of Principle. J Nucl Med. 2018;59:1092-1098.
Wallberg H, Orlova A. Slow internalization of anti-HER2 synthetic affibody monomer 111In-DOTA-ZHER2: 342-pep2: implications for development of labeled tracers. Cancer Biother Radiopharm. 2008;23:435-442.

## Claims

1. A therapeutic conjugate comprising a fusion protein and a cytotoxic radionuclide, which cytotoxic radionuclide is bound to the fusion protein, wherein:
- the fusion protein comprises and an HER2-binding region (HBR), an albumin-binding region (ABR) and a spacer region;
- the number of amino acids of the spacer region is at least 7, such as 7-30;
- the HBR comprises an amino acid sequence selected from
i) LAX₃AKX₆TX₈X₉Y HLX₁₃X₁₄X₁₅GVX₁₈DX₂₀ YKX₂₃LIDKX₂₈KT VEX₃₃VX₃₅AX₃₇YX₃₉X₄₀ ILX₄₃ALP, wherein, independently of each other,
X₃ is selected from A, G, P, S and V;
X₆ is selected from D and E;
X₈ is selected from A and V;
X₉ is selected from L and N;
X₁₃ is selected from D and T;
X₁₄ is selected from K and R;
X₁₅ is selected from I, L, M, T and V;
X₁₈ is selected from S and A;
X₂₀ is selected from F, Y and A;
X₂₃ is selected from D and R;
X₂₈ is selected from A and V;
X₃₃ is selected from G, S and D;
X₃₅ is selected from K, M and R;
X₃₇ is selected from L and R;
X₃₉ is selected from A, F and L;
X₄₀ is selected from A and E; and
X₄₃ is selected from A, H, K, P, R, T, Q and Y;
and ii) an amino acid sequence which has at least 95% identity to the sequence defined in i); and
- the ABR comprises an amino acid sequence selected from
a) LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILX₄₃X₄₄LP, wherein, independently of each other,
X₃ is selected from E, S, Q and C;
X₆ is selected from E, S, V and C;
X₇ is selected from A, L and S;
X₉ is selected from L and N;
X₁₀ is selected from A, S and R;
X₁₄ is selected from A, S, C and K;
X₂₀ is selected from Y and F;
X₂₃ is selected from N, D and R;
X₂₆ is selected from N, D and E;
X₂₇ is selected from N and K;
X₃₅ is selected from K and E;
X₃₈ is selected from I and K;
X₃₉ is selected from D, E and L;
X₄₀ is selected from A, E and H;
X₄₃ is selected from A and K;
X₄₄ is selected from A, S and E;
L in position 45 is present or absent; and
P in position 46 is present or absent;
and b) an amino acid sequence which has at least 95% identity to the sequence defined in a).

2. The therapeutic conjugate according to claim 1, wherein the cytotoxic radionuclide is coupled to a terminal end of the fusion protein, such as the C-terminal end of the fusion protein.

3. The therapeutic conjugate according to claim 1 or 2, wherein the spacer region connects the C-terminal end of the HBR to the N-terminal end of the ABR.

4. The therapeutic conjugate according to any one of the preceding claims, wherein the ABR comprises the amino acid sequence
LAX₃AKX₆X₇AX₉X₁₀ ELDX₁₄YGVSDX₂₀ YKX₂₃LIX₂₆X₂₇AKT VEGVX₃₅ALX₃₈X₃₉X₄₀ ILAALP, wherein, independently of each other,
X₃ is selected from E and S;
X₆ is selected from E and V;
X₇ is selected from A and L;
X₉ is selected from L and N;
X₁₀ is selected from A and R;
X₁₄ is selected from A, S, C and K, preferably from A, S and K;
X₂₀ is selected from Y and F;
X₂₃ is selected from N, D and R;
X₂₆ is selected from N and D;
X₂₇ is selected from N and K;
X₃₅ is selected from K and E;
X₃₈ is selected from I and K;
X₃₉ is selected from D and L;
X₄ₒ is selected from A, E and H;
L in position 45 is present or absent; and
P in position 46 is present or absent.

5. The therapeutic conjugate according to any one of the preceding claims, wherein the ABR comprises an amino acid sequence selected from the group consisting of: 
LAEAKVLANR ELDKYGVSDF YKRLINKAKT VEGVEALKLH ILAALP (SEQ ID NO:1);
LAEAKEAANA ELDSYGVSDF YKRLIDKAKT VEGVEALKDA ILAALP (SEQ ID NO:2);
GLAEAKEAAN AELDSYGVSD FYKRLIDKAK TVEGVEALKD AILAALP (SEQ ID NO:3);
LAEAKVLANR ELDKYGVSDY YKNLINNAKT VEGVKALIDE ILAALP (SEQ ID NO:4);
 and
LAEAKVLALR ELDKYGVSDY YKDLIDKAKT VEGVKALIDE ILAALP (SEQ ID NO:5).

6. The therapeutic conjugate according to any one of the preceding claims, wherein, in amino acid sequence i):
X₃ is selected from A, G, P;
X₆ is E;
X₉ is L;
X₁₃ is D;
X₁₄ is R;
X₁₅ is selected from L and V;
X₁₈ is selected from S and A;
X₂₀ is selected from F, Y and A;
X₂₈ is A;
X₃₃ is G;
X₃₅ is selected from K and R;
X₃₇ is L;
X₃₉ is selected from F and L;
X₄₀ is E; and
X₄₃ is selected from H, P and R.

7. The therapeutic conjugate according to any one of claims 1-5, wherein the HBR comprises an amino acid sequence selected from the group consisting of: 
LAAAKETALY HLDRLGVADA YKDLIDKAKT VEGVKARYFE ILHALP (SEQ ID NO:6);
LAAAKETALY HLDRVGVSDY YKDLIDKAKT VEGVRALYLE ILPALP (SEQ ID NO:7);
LAPAKETALY HLDRVGVSDY YKDLIDKAK TVEGVRALYFE ILHALP (SEQ ID NO:8);
LAAAKETALY HLDRLGVSDY YKDLIDKAK TVEGVKALYFE ILHALP (SEQ ID NO:9);
LAPAKETALY HLDRLGVSDY YKDLIDKAK TVEGVRALYLE ILKALP (SEQ ID NO:10);
LAGAKETALY HLDRLGVSDY YKDLIDKAK TVEGVRALYLE ILTALP (SEQ ID NO:11);
LAPAKETALY HLDRLGVSDY YKDLIDKAK TVEGVRALYFE ILRALP (SEQ ID NO:12);
LAGAKETALY HLDRVGVSDY YKDLIDKAK TVEGVRALYLE ILRALP (SEQ ID NO:13);
LAAAKETALY HLDRVGVSDY YKDLIDKAK TVEGVMALYAE ILPALP (SEQ ID NO:14);
LAGAKETALY HLDKTGVSDY YKDLIDKAK TVEGVRALYLE ILQALP (SEQ ID NO:15);
LAAAKETALY HLTRVGVSDY YKDLIDKAK TVEGVRALYFE ILYALP (SEQ ID NO:16);
 and
LASAKDTALY HLDRVGVSDYYKDLIDKAK TVEGVRALYAE ILAALP (SEQ ID NO:17).

8. The therapeutic conjugate according to any one of the preceding claims, wherein the cytotoxic radionuclide is selected from the group consisting of ¹⁷⁷Lu, ⁹⁰Y, ¹⁸⁸Re; ¹⁸⁶Re;¹⁶⁶Ho, ¹⁵³Sm, ⁶⁷Cu, ⁶⁴Cu/¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.

9. The therapeutic conjugate according to any one of the preceding claims, wherein the radionuclide is a radiometal and the fusion protein is conjugated to the radiometal by a chelator that is covalently bound to the fusion protein.

10. The therapeutic conjugate according to any one of the preceding claims, wherein the fusion protein comprises a cysteine (C) residue to which the radionuclide is linked, said cysteine (C) residue preferably being a terminal residue, such as a C-terminal residue.

11. The therapeutic conjugate according to claim 10, wherein the cysteine (C) residue is the only cysteine (C) residue of the fusion protein.

12. The therapeutic conjugate according to any one of the preceding claims, wherein the number of amino acids of the spacer region is at least 8, such as 8-30, such as 8-20, preferably 8-14, more preferably 10-14.

13. The therapeutic conjugate according to any one of the preceding claims, which comprises no more than 150 amino acid residues, such as no more than 130 amino acid residues.

14. A pharmaceutical composition comprising the therapeutic conjugate according to any one of the preceding claims and a pharmaceutically acceptable carrier.

15. The therapeutic conjugate according to any one of the preceding claims for use in a method of treating a cancer, such as a breast cancer or a gastric/gastroesophageal cancer.
